(19) Europäisches Patentamt
European Patent Office
Office européen des brevets

(11) Publication number : **0 349 247 B1**

(12) # EUROPEAN PATENT SPECIFICATION

(45) Date of publication of patent specification : **11.11.92 Bulletin 92/46**

(21) Application number : **89306480.8**

(22) Date of filing : **26.06.89**

(51) Int. Cl.$^5$ : **C07D 317/72,** C07D 319/08, C07D 405/06, C07D 413/06, A01N 43/30, A01N 43/32, C07D 405/04

(54) **Amino ketals, their preparation, and their use as fungicides.**

(30) Priority : **01.07.88 DK 3662/88**
**09.12.88 DK 6868/88**

(43) Date of publication of application : **03.01.90 Bulletin 90/01**

(45) Publication of the grant of the patent : **11.11.92 Bulletin 92/46**

(84) Designated Contracting States : **AT BE CH DE ES FR GB GR IT LI LU NL SE**

(56) References cited :
**EP-A- 0 281 842**
**DE-A- 1 965 321**
**CHEMICAL AND PHARMACEUTICAL BULLETIN, vol. 32, no. 3, 1984, pages 967-975; S. SUGAI et al.: "Studies on spasmolytics. I.Synthesis and spasmolytic activities of 4-acyloxy-1-(1,3-dioxolan-4-ylmethyl)piperidines"**

(56) References cited :
**CHEMICAL ABSTRACTS, vol. 90, 1979, page 508, abstract no. 72094q, Columbus, Ohio, US; J. WOLINSKI et al.: "Search foranticholinergic compounds. VII. Synthesis of 2-aminomethyl-, 4-aminomethyl-, and 2,4-bis(aminomethyl)-1,3-dioxolanes", & ACTAPOL. PHARM. 1978, 35(3), 265-72**
**CHEMICAL ABSTRACTS, vol. 88, 1978, page 586, abstract no. 6859a, Columbus, Ohio, US; & JP-A-77 83 763 (OTA PHARMACEUTICALCO., LTD) 12-07-1977**

(73) Proprietor : **CHEMINOVA AGRO A/S**
**P.O. Box 9**
**DK-7620 Lemvig (DK)**

(72) Inventor : **Svendsen, Axel**
**No. 6, Norrevang**
**DK-7620 Lemvig (DK)**
Inventor : **Wengel, Anita**
**No. 6, Norrevang**
**DK-7620 Lemvig (DK)**

(74) Representative : **Nordenbaek, Torben et al**
**c/o INTERNATIONALT PATENT-BUREAU**
**Hoeje Taastrup Boulevard 23**
**DK-2630 Taastrup (DK)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The present invention relates to novel amino ketals, a process for their preparation, and their use as fungicides.

The novel amino ketals now provided have the general formula I

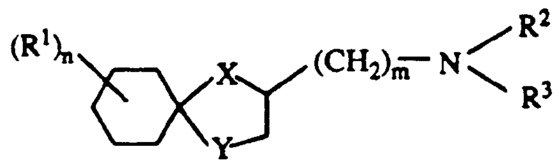

I

wherein X and Y each independently represents O or $-OCH_2-$,
$(R^1)_n$ represents up to four substituents which are the same or different and selected from straight or branched chain alkyl having up to 10 carbon atoms, cycloalkyl having up to 6 carbon atoms, aryl, aryloxy, alkoxy, cycloalkoxy, arylalkyl and alkylthio, where two adjacent $R^1$-substituents may also together form a saturated or unsaturated ring, and
n is 1, 2, 3 or 4, and m is 0, 1, 2, 3 or 4, and
$R^2$ and $R^3$ each independently represents hydrogen, straight or branched chain alkyl having up to 10 carbon atoms, unsubstituted or alkylsubstituted cycloalkyl, alkenyl,alkynyl or unsubstituted or alkylsubstituted arylalkyl, or
$R^2$ and $R^3$ when taken together with the nitrogen atom to which they are attached form a saturated or unsaturated, substituted or unsubstituted heterocyclic ring which may optionally be aryl-fused or cycloalkyl fused and which may optionally contain one or more additional hetero atoms selected from O and N,
with the proviso that if X and Y are both O, m is 1, and $-NR^2R^3$ is other than an unsaturated, substituted or unsubstituted heterocyclic ring which may optionally be aryl-fused or cycloalkyl-fused and which may optionally contain one or more additional hetero atoms selected from O and N, then $(R^1)_n$ cannot be a sole substituent (n=1) in the 4-position in the cyclohexane ring and of the formula

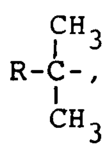

wherein R represents hydrogen, alkyl or optionally substituted phenyl, and with the further proviso that if X and Y are both O, m is 1, and $-NR^2R^3$ is amino or phthalimido, then $(R^1)_n$ cannot be solely 8-methyl, 8-methoxy or 6-phenyl or solely 7,7,9,9-tetramethyl.

The symbols $R^1$-$R^3$ in the above formula I have preferably, or by way of example, the following meanings:

$R^1$: alkyl is preferably alkyl having 4-10 carbon atoms, more preferably branched chain alkyl having 4-8 carbon atoms,

aryl is preferably phenyl,

aryloxy is preferably phenoxy,

alkoxy is, e.g., methoxy or isopropoxy,

arylalkyl is, e.g., 1-methyl-1-phenylethyl,

alkyl in alkylthio is preferably alkyl having up to 12 carbon atoms,

two adjacent $R^1$-substituents which together form a saturated or unsaturated ring are preferably fused cyclohexyl or fused phenyl, respectively,

$R^2$ and $R^3$: straight and branched chain alkyl is preferably alkyl having up to 6 carbon atoms and more preferably up to 4 carbon atoms,

cycloalkyl is preferably cyclohexyl,

alkenyl is preferably allyl,

alkynyl is preferably propargyl,

arylalkyl is preferably benzyl,

the optionally substituted heterocyclic ring is preferably piperidino, mono- or dimethylpiperidino, ethyl-

piperidino, ethylmethylpiperidino, phenylpiperidino, morpholino, or 2,6-dimethylmorpholino (cis/trans or cis or trans),

aryl in aryl-fused heterocyclic ring is preferably phenyl, and

cycloalkyl in cycloalkyl-fused heterocyclic ring is preferably cyclohexyl.

Compounds disclosed in GB-A-1,197,313 are disclaimed by the proviso in the present application. The compounds of this prior art are stated to be pharmaceutically acceptable and have antihypertensive activity.

Compounds of formula I can exist as geometrical and/or optical isomers or isomer mixtures of varying composition. The present invention relates to both the pure isomers and isomer mixtures.

Furthermore, it has been found that the novel amino ketals of the general formula I, wherein X and Y, the substituents $R^1$-$R^3$ and n and m have the above defined meanings, are obtainable by reacting a ketal of the general formula

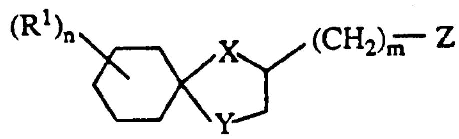

II

wherein X, Y, $(R^1)_n$ and m have the above defined meanings, and wherein Z represents an exchangeable electron-attracting group as, e.g., a halogen atom or a methanesulphonyloxy group or a arylsulphonyloxy group, with an amine of the general formula

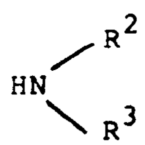

III

wherein $R^2$ and $R^3$ have the above defined meanings, optionally in the presence of a diluent and optionally in the presence of a catalyst, and optionally in the presence of an acid-binding agent in addition to a possible excess of the amine III.

The ketals of formula II, e.g. when Z is Cl and both of X and Y are oxygen, are obtainable in a generally known manner by reacting a ketone of the formula

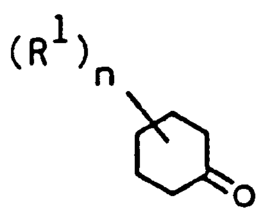

IV

wherein $(R^1)_n$ has the above defined meaning, with epichlorohydrin of the formula

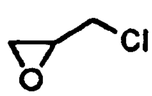

V

in a manner known per se in the presence of a diluent, e.g., methylene chloride, and in the presence of a catalyst (confer what is stated in React. Kinet. Catal. Lett. 1981, 17(3-4), 281-6).

It has been found that the novel amino ketals of formula I have fungicidal properties.

The active compounds of the invention can be used in practice for combating phytopathogenic fungi as for example Plasmodiophoromycetes, Chytridiomycetes, Zygomycetes, Ascomycetes, Basidiomycetes and Deuteromycetes.

The active substances of the invention can be used, e.g., for combating the plant diseases Puccinia recondita, Puccinia striiformis and other rust diseases on wheat, Puccinia hordei, Puccinia striiformis and other

rust diseases on barley and rust on other host plants as for example coffee, apples, vegetables and ornamental plants.
Erysiphe graminis (mildew) on barley and wheat and other true mildew species on various host plants such as Sphaerotheca fuliginea on cucumber, Podosphaera leucotricha on apples and Uncinula necator on vines.
Helminthosporium spp., e.g. Pyrenophora teres (net blotch) on barley.
Rhynchosporium spp. and Pseudocercosporella herpotrichoides on cereals.
Cercospora arachidicola on peanuts and other Cercospora species on for example sugar beet, banana and soya bean.
Botrytis cinerea (grey mold) on tomato, strawberry, vine and other host plants.
Venturia inaequalis (scab) on apples.
Pyricularia oryzae on rice.

The active substances are active in vitro against a broad spectrum of fungi.

The active substances are active as seed dressing agents against Fusarium spp., Septoria spp., Tilletia spp., Ustilago spp., Helminthosporium spp. and Pseudocercosporella herpotrichoides on cereals, Rhizoctonia solani on cotton and Corticium sasakii on rice.

Besides an excellent curative activity the active substances of the invention also show extremely good protective properties as well as systemic activity.

In addition to this some of the compounds are sufficiently volatile to be active in the gaseous phase against fungi on plants.

The compounds may also be useful as industrial (as opposed to agricultural) fungicides, e.g. in the prevention of fungal attack on wood, hides, leather and especially paint films.

The compounds are also useful for the treatment of human fungal infections as for example candidiasis and dermatophyte infections.

In accordance with the above the invention also relates to a fungicidal composition, particularly a plant-fungicidal composition, and more particularly a cereal-fungicidal composition for combating mildew, rust and other significant foliage diseases, which composition is characterised by containing as an active component at least one compound selected from the amino ketals of the above formula I.

Moreover, the invention relates to the use of an amino ketal of the above formula I for combating fungi, particularly phytopathogenic fungi, and more particularly mildew, rust and other significant foliage diseases on cereals.

Furthermore, the invention relates to a method of combating fungi, particularly phytopathogenic fungi, and more particularly mildew, rust and other significant foliage diaseses on cereals which method is characterised by allowing an amino ketal of the above formula I to affect the fungi concerned and/or their biotope.

The compounds can be applied in a number of ways. For example they can be applied, formulated or unformulated, directly to the foliage of a plant, or they can be applied to bushes and trees, to seeds or to other media in which plants, bushes or trees are growing or are to be planted, or they can be sprayed on, dusted on or applied as a cream or paste formulation, or they can be applied as a vapour, or as slow release granules. Application can be to any part of the plant, bush or tree, for example to the foliage, stems, branches or roots, or to soil surrounding the roots, or to the seed before it is planted, or to soil generally, to paddy water or to hydroponic culture systems. The compounds of the invention may also be injected into plants or trees and they may also be sprayed onto vegetation using electrodynamic spraying techniques.

Furthermore, the invention relates to a process of preparing a fungicidal composition, particularly a plant-fungicidal composition, and more particularly a cereal-fungicidal composition for combating mildew, rust and other significant foliage diseases, which process is characterised by mixing an amino ketal of the above formula I with one or more agents selected from extending agents, surface active agents and other conventional auxiliary agents.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed. Alternatively the active ingredient can be formulated for seed dressing purposes using an organic solvent (for example N-methylpyrrolidone or dimethylformamide).

The compositions may also be in the form of dispersible powders or granules comprising a wetting agent to facilitate the dispersion in liquids of the powder or granules which may contain also fillers and suspending agents.

The aqueous dispersions or emulsions may be prepared by dissolving the active ingredient(s) in an organic solvent optionally containing wetting, dispersing or emulsifying agents and then adding the mixture to water which may also contain wetting, dispersing or emulsifying agents. Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating (for example coated with) the compound are preferred. Such granules suitably contain up to 25% by weight of the compound. The invention therefore also provides a fertiliser composition comprising the compound of general formula I.

The compositions may also be in the form of liquid preparations for use as dips or sprays which are generally aqueous dispersions or emulsions containing the active ingredient in the presence of one or more surfactants, e.g. wetting agents, dispersing agents, emulsifying agents or suspending agents, or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzene-sulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homo geneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (e.g. alkyl or aryl sulphonic acids such as xylenesulphonic acid or dodecyl benzenesulphonic acid) since the presence of such acids can increase the solubility of the active ingredients in the polar solvents often used in the concentrates. The concentrates suitably contain also a high proportion of surfactants so that sufficiently stable emulsions in water can be obtained. After dilution to form aqueos preparations, such preparations may contain varying amounts of the active ingredients depending upon the intended purpose, but an aqueous preparation containing 0.0005% or 0.01% to 10% by weight of active ingredient may be used.

The compositions of this invention can comprise also other compounds having biological activity, e.g. compounds having similar or complementary fungicidal activity or compounds having plant growth regulating, herbicidal or insecticidal activity.

The other fungicidal compound can be, for example, one which is capable of combating ear diseases of cereals (e.g. wheat) such as Septoria, Gibberella and Helminthosporium spp., seed and soil borne diseases and downy and powdery mildews on vines and powdery mildew and scab on apples etc. These mixtures of fungicides can have a broader spectrum of activity than the compound of general formula I alone. Furthermore, the other fungicide can have a synergistic effect on the fungicidal activity of the compound of general formula I.

Examples of the other fungicidal compound are carbendazim, benomyl, thiophanate-methyl, thiabendazole, fuberidazol, etridazol, dichlofluanid, cymoxanil, oxadixyl, ofurace, metalaxyl, furalaxyl, benalaxyl, fosetyl-aluminium, fenarimol, iprodion, procymidion, vinclozolin, penconazol, myclobutanil, R0151297, S3308, pyrazophos, ethirimol, ditalimfos, tridemorph, triforin, nuarimol, triazbutyl, guazatin, triacetate salt of 1,1'-iminodi(octamethylene)-diguanidine, propiconazol, prochloraz, flutriafol, hexaconazol, i.e. the compound 1-(1,2,4-triazol-1-yl)-2-(2,4-dichlorophenyl)hexan-2-ol, DPX H6573 (1-((bis-4-fluorophenyl)methyl silyl)methyl-1H-1,2,4-triazol, triadimefon, triadimenol, diclobutrazol, fenpropimorph, pyrifenox, (2RS,3RS)-2-(4-chlorophenyl)-3-cyclopropyl-1-(1H-1,2,4-triazol-1-yl)butan-2-ol, (RS)-1-(4-chlorophenyl-4,4-dimethyl-3-(1H-1,2,4-triazol-1-ylmethyl)-pentan-3-ol, 4-chloro-N-(cyano(ethoxy)methyl)benzamide, fenpropidin, chlorozolinate, diniconazol, imizalil, fenfuram, carboxin, oxycarboxin, methfuroxam, dodemorph, BAS 454, blasticidin S, kasugamycin, edifenphos, kitazin P, cycloheximide, phthalide, probenazol, isoprothiolan, tricyclazol, pyroquilon, chlorbenzthiazon, neoasozin, polyoxin D, validamycin A, mepronil, flutolanil, pencycuron, diclomezin, phenazineoxide, nickel dimethyldithiocarbamate, techlofthalam, bitertanol, bupirimat, etaconazol, streptomycin, cypofuram, biloxazol, quinomethionate, dimethirimol, 1-(2-cyano-2-methoxyiminoacetyl)-3-ethyl urea, fenapanil, tolclofosmethyl, pyroxyfur, polyram, maneb, mancozeb, captafol, chlorothalonil, anilazin, thiram, captan, folpet, zineb, propineb, sulphur, dinocap, binapacryl, nitrothalisopropyl, dodine, dithianon, fentin hydroxide, fentin acetate, tecnazene, quintozen, dichloran, copper-containing compounds such as copper oxychloride, copper sulphate and Bordeaux mixture as well as organic mercury compounds.

The compounds of general formula I can be mixed with soil, peat or other rooting media for the protection of the plants against seed-borne, soil-borne or foliar fungal diseases.

Suitable insecticides that can be incorporated in the composition of the invention include pirimicarb, dimethoate, demeton-s-methyl, formothion, carbaryl, isoprocarb, XMC, BPMC, carbofuran, carbosulfan, diazinon, fenthion, fenitrothion, phenthoate, chlorpyrifos, isoxathion, propaphos, monocrotophos, buprofezin, ethoproxyfen and cycloprothrin.

Plant growth regulating compounds for use in compositions of the invention are compounds which control weeds or seedhead formation or selectively control the growth of less desirable plants (e.g. grasses).

Examples of suitable plant growth regulating compounds for use with the compositions of the invention are the gibberellins (e.g. $GA_3$, $GA_4$ or $GA_7$), the auxins (e.g. indoleacetic acid, indolebutyric acid, naphthoxyacetic acid or naphthylacetic acid), the cytokinins (e.g. kinetin, diphenylurea, benzimidazole, benzyladenine or benzylaminopurine), phenoxyacetic acids (e.g. 2,4-D or MCPA), substituted benzoic acids (e.g. triiodobenzoic acid), morphactins (e.g. chlorfluorecol), maleic hydrazide, glyphosate, glyphosine, long chain fatty alcohols and acids, dikegulac, paclobutrazol, fluorprimidol, fluoridamid, mefluidide, substituted quaternary ammonium and phosphonium compounds (e.g. chlormequat, chlorphonium or mepiquatchloride), ethephon, carbetamide, methyl-3,6-dichloroanisate, daminozide, asulam, abscisic acid, isopyrimol, 1-(4-chlorophenyl)-4,6-dimethyl-2-oxo-1,2-dihydropyridine-3-carboxylic acid, hydroxybenzonitriles (e.g. bromoxynil), difenzoquat, benzoylprop-ethyl 3,6-dichloropicolinic acid, fenpentezol, inabenfid, triapenthenol, and tecnazene.

The following Examples illustrate the invention.

## PREPARATION EXAMPLE 1

Preparation of 2-(N-1,2,5,6-tetrahydropyridylmethyl)-8-(1,1-dimethylethyl)-1,4-dioxaspiro[4,5]decane, compound No. 35.

A mixture of 2-chloromethyl-8-(1,1-dimethylethyl)-1,4-dioxaspiro[4,5]decane (3.00 g, 0.012 mol), 1,2,5,6-tetrahydropyridine (3.00 g, 0.036 mol) potassium iodide (0.6 g) and dimethylsulfone (4.8 g) is heated on an oil bath at 150°C for three hours. The reaction mixture is cooled and poured into water (150 ml) and then extracted with ether (100 ml). The ether phase is separated, dried over sodium sulphate and evaporated in vacuo. The resulting oil is purified by kugelrohr distillation. Yield 2.80 g (78%) of a viscous oil of boiling point 180°C/0.01 mm Hg; $n_D^{26}$ : 1.4911.

## PREPARATION EXAMPLE 2

Preparation of 2-(2-N,N-dipropylaminoethyl)-8-(1,1-dimethylethyl)-1,4-dioxaspiro[4,5]decane, compound No. 88.

A mixture 2-(2-methanesulphonyloxyethyl)-8-(1,1-dimethylethyl)-1,4-dioxaspiro[4,5]decane (2.30 g, 0,0072 mol), N,N-dipropylamine (2.02 g, 0.01 mol), potassium iodide (0.2 g) and potassium carbonate (2.0 g) dissolved in dimethylformamide (15 ml) is heated under reflux for three hours. The reaction mixture is cooled

and poured into water (150 ml) and then extracted with ether (100 ml). The ether phase is separated, dried over sodium sulphate and evaporated in vacuo. The resulting oil is purified by kugelrohr distillation. Yield 1.54 g (66%) of a viscous oil of boiling point 220°C/10 mm Hg; $n_D^{26}$ : 1.4661.

## PREPARATION EXAMPLE 3

Preparation of 3-(4-methylpiperidinomethyl-9-(1,1-dimethylethyl)-1,5-dioxaspiro[5,5]undecane, compound No. 98.

A mixture of 3-methanesulphonyloxymethyl-9-(1,1-dimethylethyl)-1,5-dioxaspiro[5,5]undecane (2.56 g, 0.008 mol), 4-methylpiperidine (0.99 g, 0.01 mol), potassium iodide (0.2 g) and potassium carbonate (2.5 g) dissolved in dimethylformamide (15 ml) is heated under reflux for three hours. The reaction mixture is cooled and poured into water (150 ml) and then extracted with ether (100 ml). The ether phase is separated, dried over sodium sulphate and evaporated in vacuo. The resulting oil is purified by kugelrohr distillation. Yield 1.94 g (75%) of a viscous oil of boiling point 160°C/0.04 mm Hg. The oil crystallizes on standing to a solid of melting point 75-77°C.

The amino ketals of formula I according to the invention are exemplified by specific examples of compounds as stated in the following Table 1 wherein the term Refractive Index (26C) is identical to Refractive Index $n_D^{26}$.

Table 1

| | | |
|---|---|---|
| No. 1 | 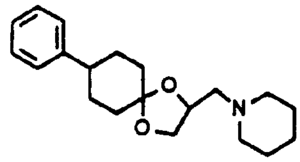 | Refractive Index (26 C) 1.5266 |
| No. 2 | 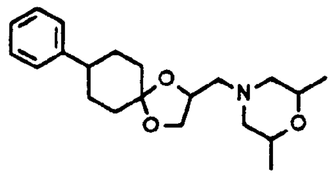 | Refractive Index (26 C) 1.5205 |
| No. 3 | 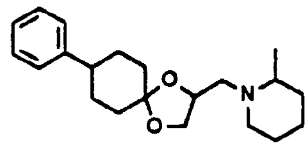 | Refractive Index (26 C) 1.5255 |
| No. 4 | 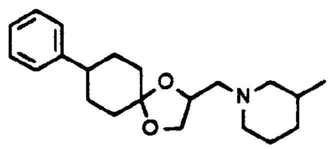 | Refractive Index (26 C) 1.5213 |
| No. 5 | 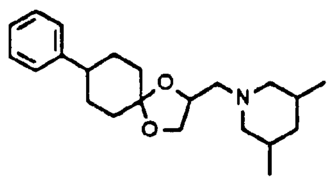 | Refractive Index (26 C) 1.5167 |
| No. 6 | 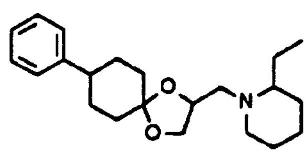 | Refractive Index (26 C) 1.5244 |
| No. 7 | 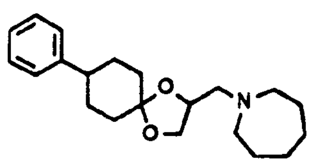 | Refractive Index (26 C) 1.5250 |
| No. 8 | 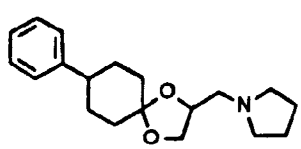 | Refractive Index (26 C) 1.5299 |

| No. | | |
|---|---|---|
| 9 | 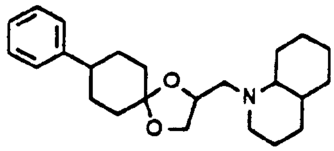 | Refractive Index (26 C) 1.5358 |
| 10 | 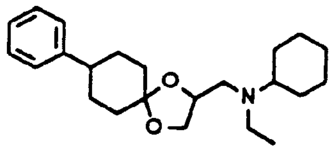 | Refractive Index (26 C) 1.5218 |
| 11 | 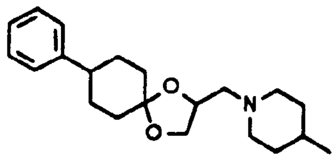 | Refractive Index (26 C) 1.5201 |
| 12 | 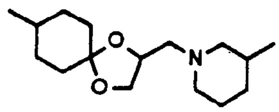 | Refractive Index (26 C) 1.4753 |
| 13 | 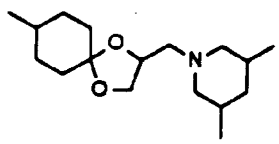 | Refractive Index (26 C) 1.4729 |
| 14 | 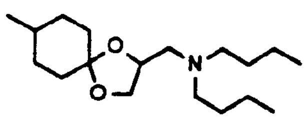 | Refractive Index (26 C) 1.4609 |
| 15 | 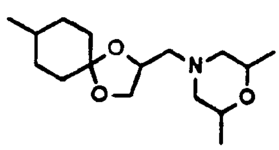 | Refractive Index (26 C) 1.4718 |
| 16 | 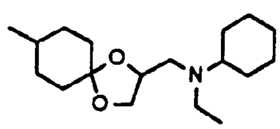 | Refractive Index (26 C) 1.4818 |

No. 17

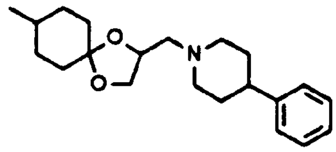

Refractive Index (26 C) 1.5223

---

No. 18

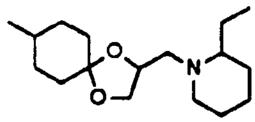

Refractive Index (26 C) 1.4815

---

No. 19

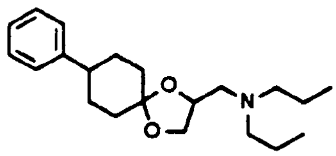

Refractive Index (26 C) 1.5082

---

No. 20

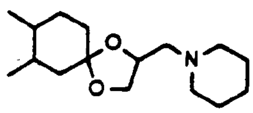

Refractive Index (26 C) 1.4821

---

No. 21

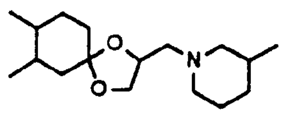

Refractive Index (26 C) 1.4788

---

No. 22

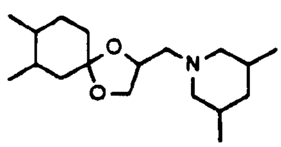

Refractive Index (26 C) 1.4762

---

No. 23

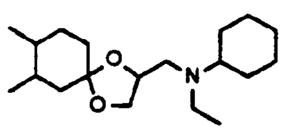

Refractive Index (26 C) 1.4835

---

No. 24

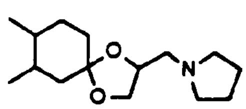

Refractive Index (26 C) 1.4810

---

No. 25

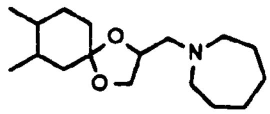

Refractive Index (26 C)

1.4849

---

No. 26

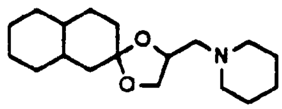

Refractive Index (26 C)

1.5026

---

No. 27

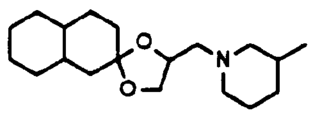

Refractive Index (26 C)

1.4977

---

No. 28

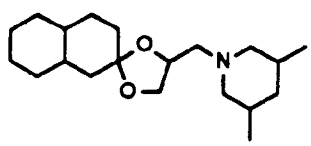

Refractive Index (26 C)

1.4950

---

No. 29

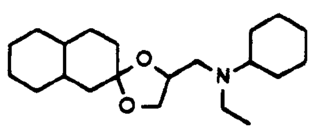

Refractive Index (26 C)

1.5005

---

No. 30

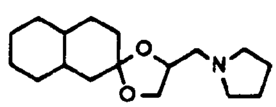

Refractive Index (26 C)

1.5028

---

No. 31

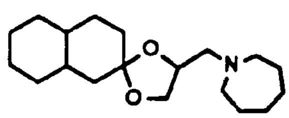

Refractive Index (26 C)

1.5021

---

No. 32

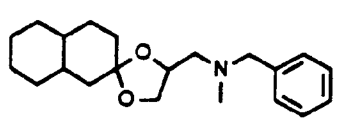

Refractive Index (26 C)

1.5308

---

No. 33

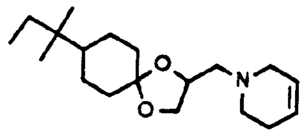

Refractive Index (26 C) 1.4940

No. 34

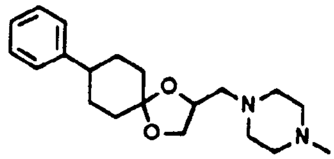

Refractive Index (26 C) 1.5304

No. 35

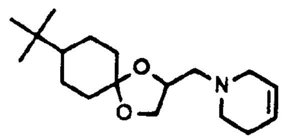

Refractive Index (26 C) 1.4911

No. 36

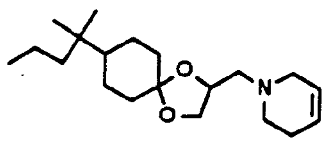

Refractive Index (26 C) 1.4924

No. 37

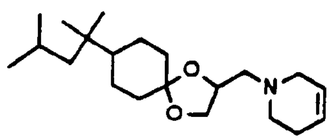

Refractive Index (26 C) 1.4915

No. 38

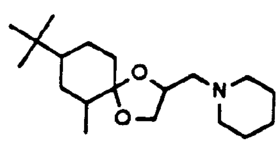

Refractive Index (26 C) 1.4806

No. 39

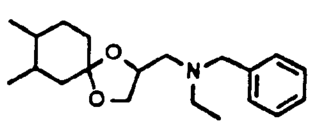

Refractive Index (26 C) 1.5151

No. 40

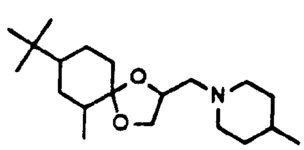

Refractive Index (26 C) 1.4769

No.
41

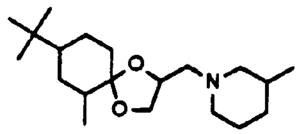

Refractive Index (26 C)
1.4780

---

No.
42

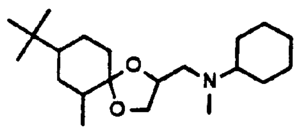

Refractive Index (26 C)
1.4850

---

No.
43

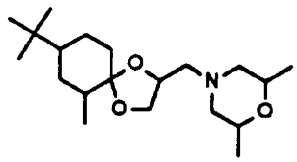

Refractive Index (26 C)
1.4751

---

No.
44

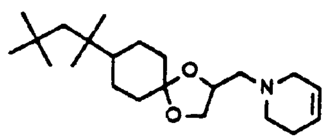

Refractive Index (26 C)
1.4934

---

No.
45

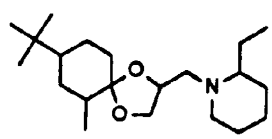

Refractive Index (26 C)
1.4832

---

No.
46

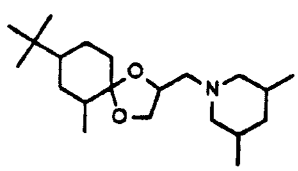

Refractive Index (26 C)
1.4763

---

No.
47

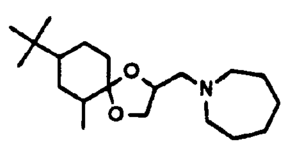

Refractive Index (26 C)
1.4850

---

No.
48

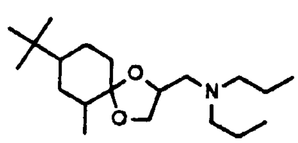

Refractive Index (26 C)
1.4650

---

No. 49

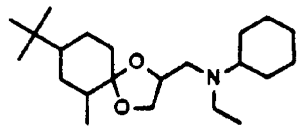

Refractive Index (26 C) 1.4831

---

No. 50

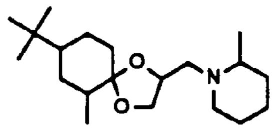

Refractive Index (26 C) 1.4812

---

No. 51

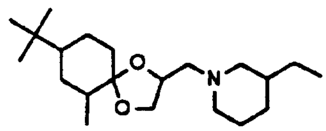

Refractive Index (26 C) 1.4794

---

No. 52

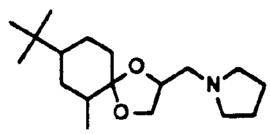

Refractive Index (26 C) 1.4808

---

No. 53

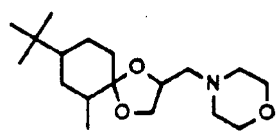

Refractive Index (26 C) 1.4818

---

No. 54

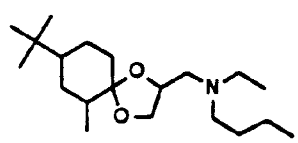

Refractive Index (26 C) 1.4666

---

No. 55

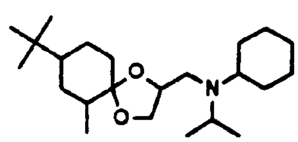

Refractive Index (26 C) 1.4836

---

No. 56

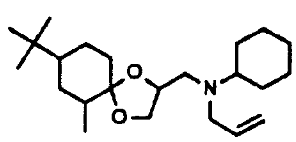

Refractive Index (26 C) 1.4887

---

| | | |
|---|---|---|
| No. 57 | 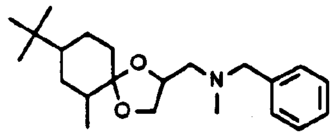 | Refractive Index (26 C) 1.5049 |
| No. 58 | 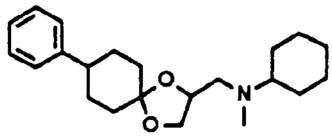 | Refractive Index (26 C) 1.5257 |
| No. 59 | 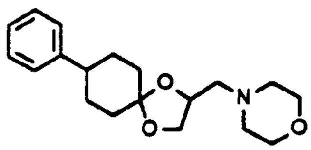 | Refractive Index (26 C) 1.5311 |
| No. 60 | 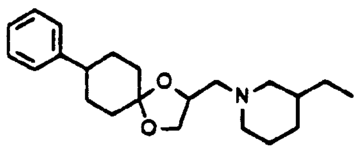 | Refractive Index (26 C) 1.5200 |
| No. 61 | 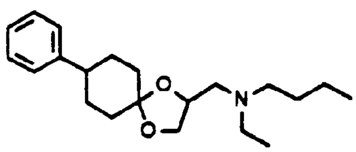 | Refractive Index (26 C) 1.5093 |
| No. 62 | 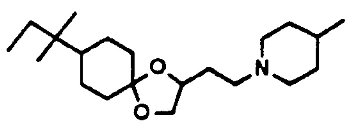 | Refractive Index (26 C) 1.4813 |
| No. 63 | 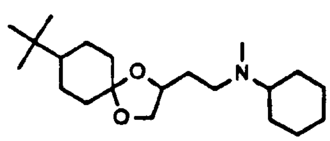 | Refractive Index (26 C) 1.4842 |
| No. 64 | 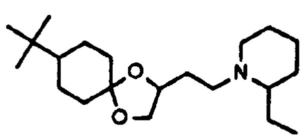 | Refractive Index (26 C) 1.4828 |

No.

65

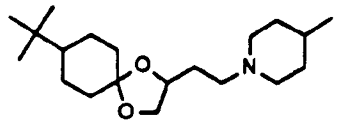

Refractive Index (26 C)

1.4789

---

No.

66

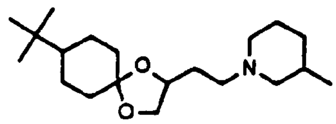

Refractive Index (26 C)

1.4800

---

No.

67

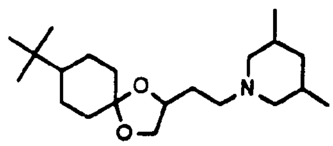

Refractive Index (26 C)

1.4788

---

No.

68

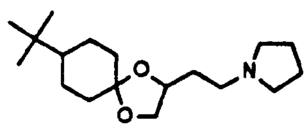

Refractive Index (26 C)

1.4810

---

No.

69

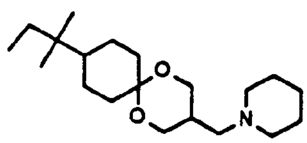

Refractive Index (26 C)

1.4903

---

No.

70

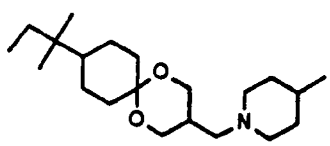

Refractive Index (26 C)

1.4868

---

No.

71

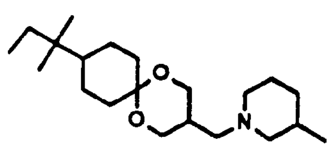

Refractive Index (26 C)

1.4872

---

No.

72

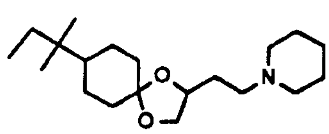

Refractive Index (26 C)

1.4851

---

| No. | | |
|---|---|---|
| 73 | 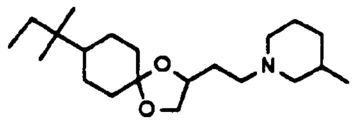 | Refractive Index (26 C) 1.4826 |
| 74 | 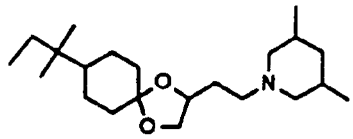 | Refractive Index (26 C) 1.4810 |
| 75 | 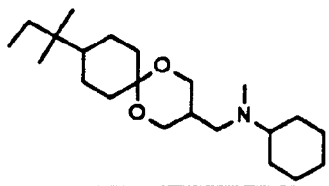 | Refractive Index (26 C) 1.4917 |
| 76 | 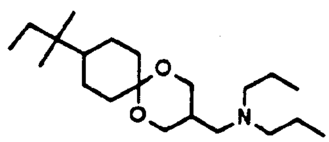 | Refractive Index (26 C) 1.4751 |
| 77 | 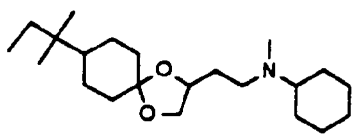 | Refractive Index (26 C) 1.4866 |
| 78 | 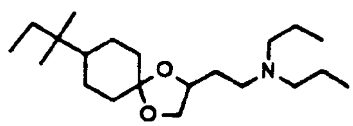 | Refractive Index (26 C) 1.4700 |
| 79 | 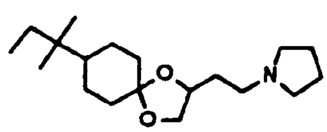 | Refractive Index (26 C) 1.4836 |
| 80 | 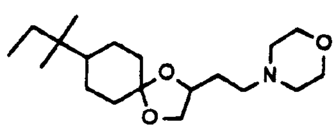 | Refractive Index (26 C) 1.4847 |

| No. | | |
|---|---|---|
| 81 | 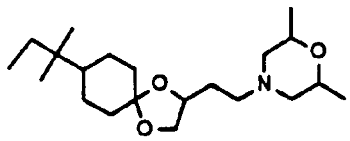 | Refractive Index (26 C)<br>1.4803 |
| 82 | 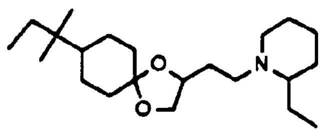 | Refractive Index (26 C)<br>1.4850 |
| 83 | 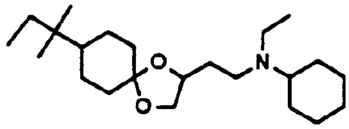 | Refractive Index (26 C)<br>1.4842 |
| 84 | 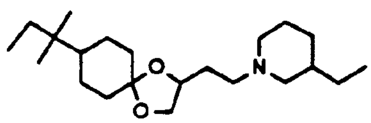 | Refractive Index (26 C)<br>1.4832 |
| 85 | 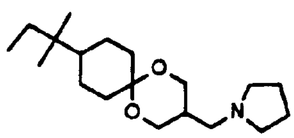 | Refractive Index (26 C)<br>1.4894 |
| 86 | 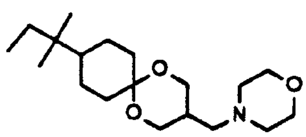 | Refractive Index (26 C)<br>1.4902 |
| 87 | 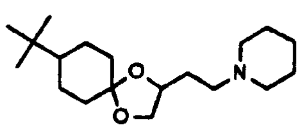 | Refractive Index (26 C)<br>1.4820 |
| 88 | 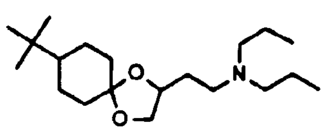 | Refractive Index (26 C)<br>1.4661 |

No.
89
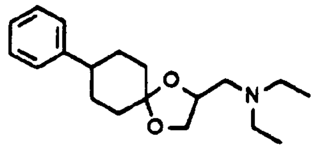
Refractive Index (26 C)
1.5130

---

No.
90
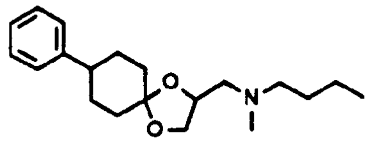
Refractive Index (26 C)
1.5106

---

No.
91
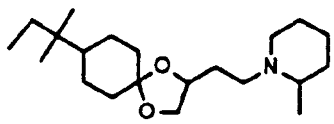
Refractive Index (26 C)
1.4813

---

No.
92
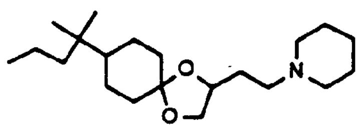
Refractive Index (26 C)
1.4846

---

No.
93
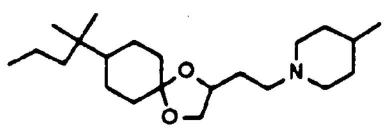
Refractive Index (26 C)
1.4814

---

No.
94
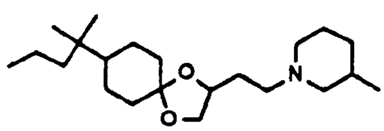
Refractive Index (26 C)
1.4822

---

No.
95
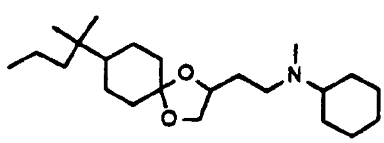
Refractive Index (26 C)
1.4862

---

No.
96
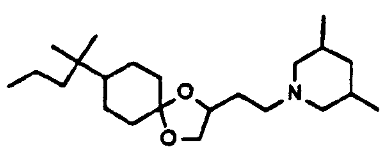
Refractive Index (26 C)
1.4811

---

No. 97

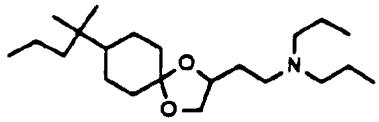

Refractive Index (26 C) 1.4701

---

No. 98

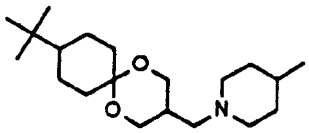

Mp 75 - 77°C

---

No. 99

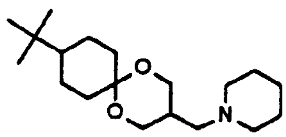

Mp 105-106°C

---

No. 100

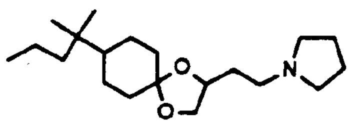

Refractive Index (26 C) 1.4835

---

No. 101

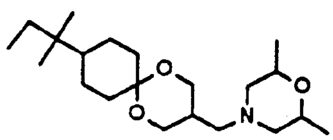

Refractive Index (26 C) 1.4851

---

No. 102

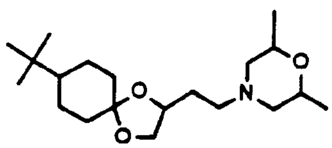

Refractive Index (26 C) 1.4768

---

No. 103

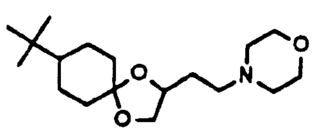

Refractive Index (26 C) 1.4823

---

No. 104

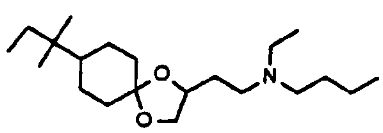

Refractive Index (26 C) 1.4703

---

No. 105

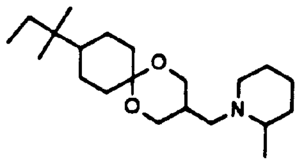

Refractive Index (26 C)

1.4893

---

No. 106

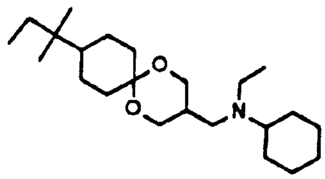

Refractive Index (26 C)

1.4911

---

No. 107

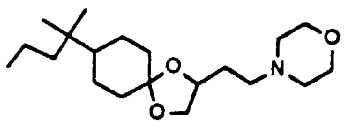

Refractive Index (26 C)

1.4845

---

No. 108

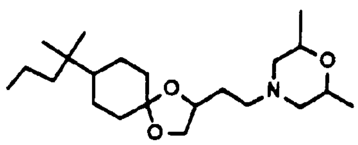

Refractive Index (26 C)

1.4797

---

No. 109

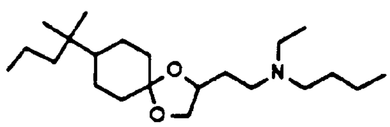

Refractive Index (26˚C)

1.4701

---

No. 110

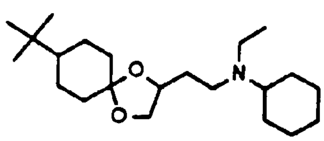

Refractive Index (26 C)

1.4822

---

No. 111

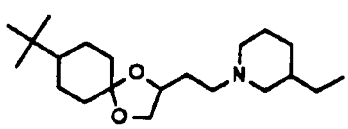

Refractive Index (26 C)

1.4804

---

No. 112

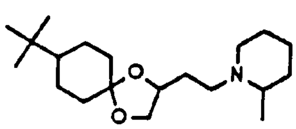

Refractive Index (26 C)

1.4818

---

No.

113

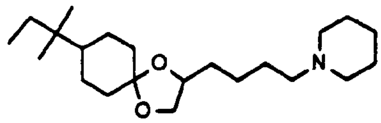

Refractive Index (26 C)

1.4836

No.

114

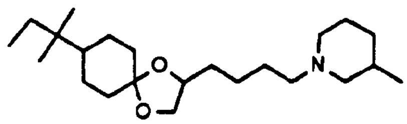

Refractive Index (26 C)

1.4814

No.

115

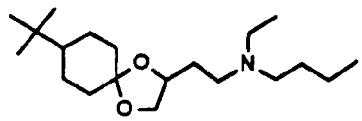

Refractive Index (26 C)

1.4678

No.

116

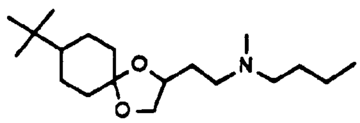

Refractive Index (26 C)

1.4677

No.

117

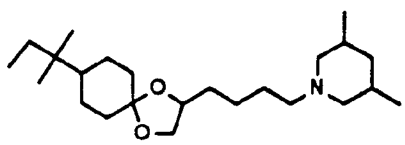

Refractive Index (26 C)

1.4803

No.

118

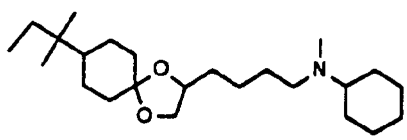

Refractive Index (26 C)

1.4852

No.

119

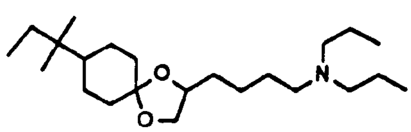

Refractive Index (26 C)

1.4702

No.

120

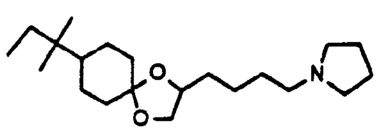

Refractive Index (26 C)

1.4825

No.

121

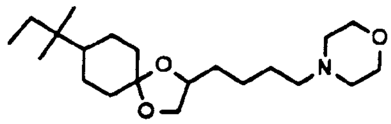

Refractive Index (26 C)

1.4834

No.

122

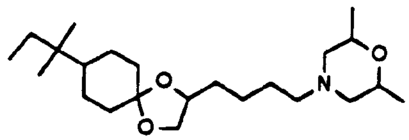

Refractive Index (26 C)

1.4790

No.

123

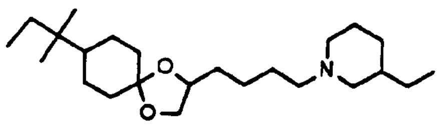

Refractive Index (26 C)

1.4822

No.

124

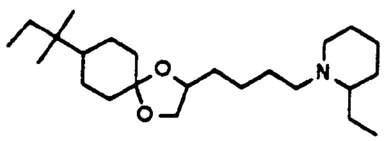

Refractive Index (26 C)

1.4840

No.

125

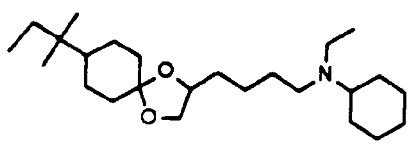

Refractive Index (26 C)

1.4833

No.

126

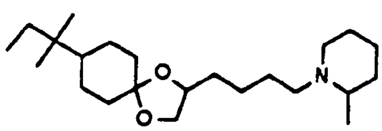

Refractive Index (26 C)

1.4831

No.

127

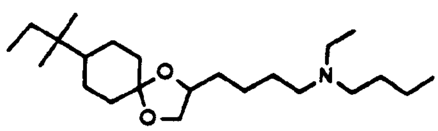

Refractive Index (26 C)

1.4701

No.

128

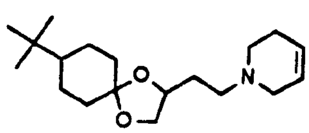

Refractive Index (26 C)

1.4903

No. 129

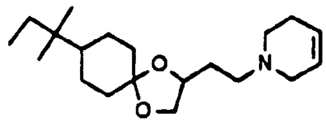

Refractive Index (26 C)

1.4924

---

No. 130

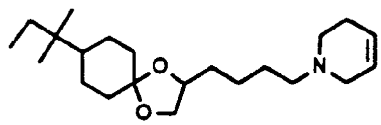

Refractive Index (26 C)

1.4911

---

No. 131

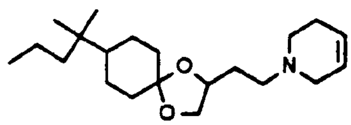

Refractive Index (26 C)

1.4917

---

No. 132

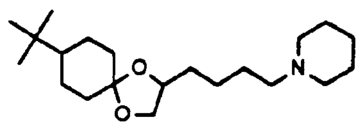

Refractive Index (26 C)

1.4814

---

No. 133

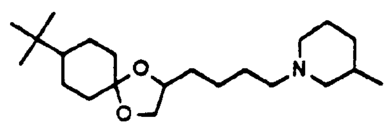

Refractive Index (26 C)

1.4789

---

No. 134

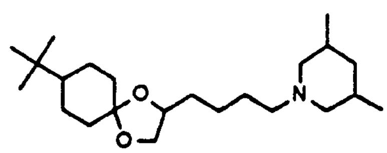

Refractive Index (26 C)

1.4776

---

No. 135

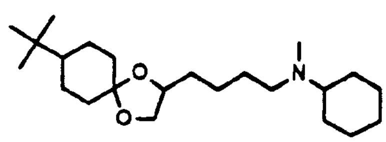

Refractive Index (26 C)

1.4830

---

No. 136

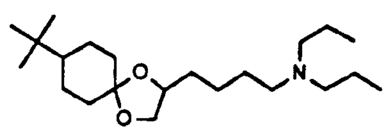

Refractive Index (26 C)

1.4667

---

No. 137

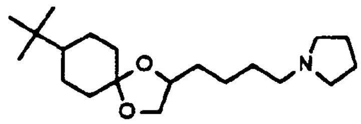

Refractive Index (26 C)

1.4801

---

No. 138

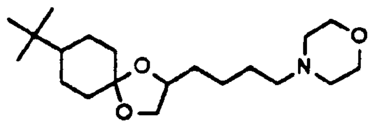

Refractive Index (26 C)

1.4808

---

No. 139

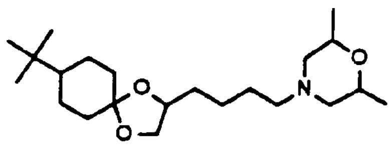

Refractive Index (26 C)

1.4762

---

No. 140

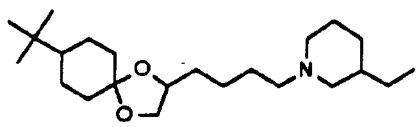

Refractive Index (26 C)

1.4796

---

No. 141

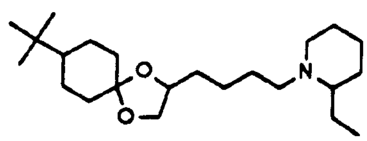

Refractive Index (26 C)

1.4812

---

No. 142

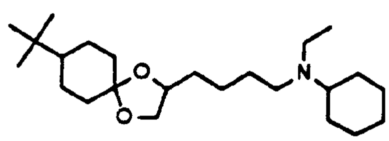

Refractive Index (26 C)

1.4802

---

No. 143

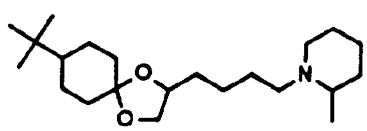

Refractive Index (26 C)

1.4804

---

No. 144

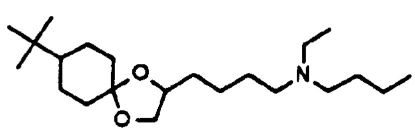

Refractive Index (26 C)

1.4670

---

No. 145

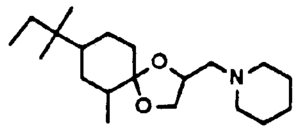

Refractive Index (26 C) 1.4846

---

No. 146

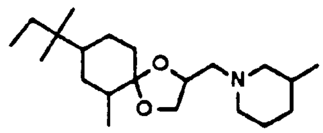

Refractive Index (26 C) 1.4815

---

No. 147

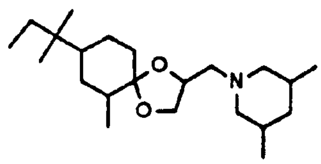

Refractive Index (26 C) 1.4795

---

No. 148

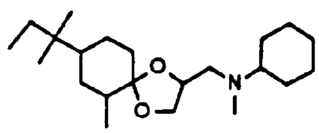

Refractive Index (26 C) 1.4877

---

No. 149

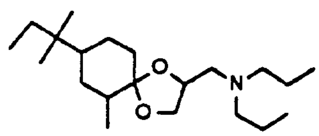

Refractive Index (26 C) 1.4690

---

No. 150

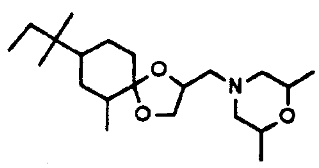

Refractive Index (26 C) 1.4788

---

No. 151

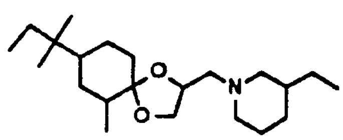

Refractive Index (26 C) 1.4827

---

No. 152

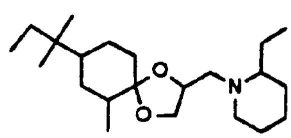

Refractive Index (26 C) 1.4858

---

No. 153

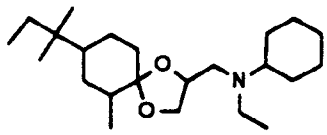

Refractive Index (26 C) 1.4853

---

No. 154

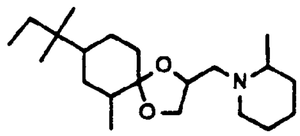

Refractive Index (26 C) 1.4848

---

No. 155

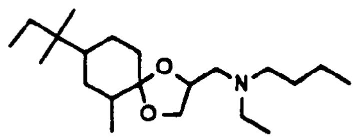

Refractive Index (26 C) 1.4697

---

No. 156

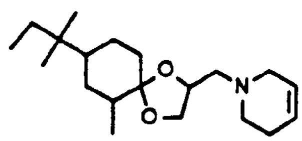

Refractive Index (26 C) 1.4938

---

No. 157

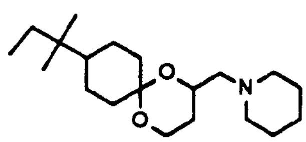

Refractive Index (26 C) 1.4907

---

No. 158

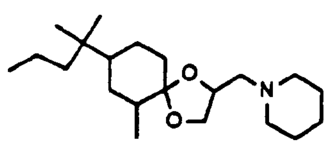

Refractive Index (26 C) 1.4842

---

No. 159

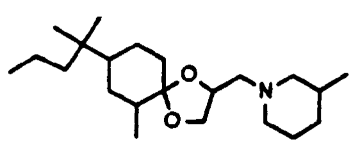

Refractive Index (26 C) 1.4810

---

No. 160

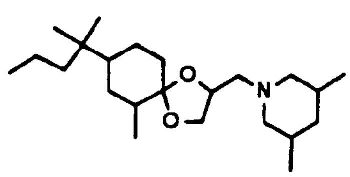

Refractive Index (26 C) 1.4795

---

No.

161

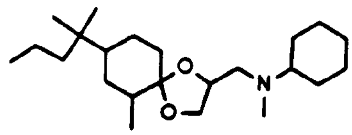

Refractive Index (26 C)
1.4897

---

No.

162

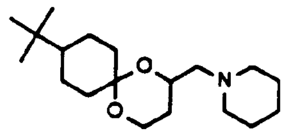

Refractive Index (26 C)
1.4867

---

No.

163

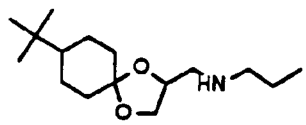

Refractive Index (26 C)
1.4679

---

No.

164

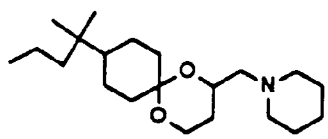

Refractive Index (26 C)
1.4890

---

No.

165

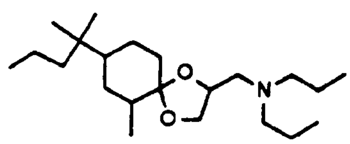

Refractive Index (26 C)
1.4688

---

No.

166

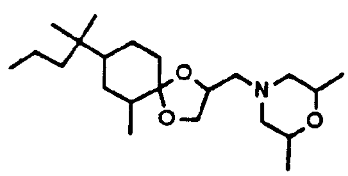

Refractive Index (26 C)
1.4782

---

No.

167

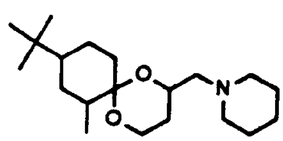

Refractive Index (26 C)
1.4847

---

No.

168

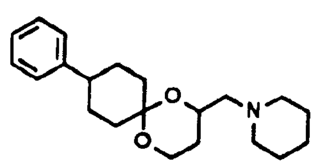

Refractive Index (26 C)
1.5299

---

No. 169

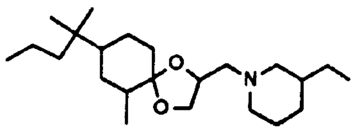

Refractive Index (26 C) 1.4821

No. 170

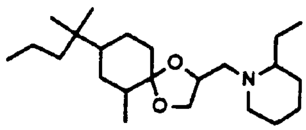

Refractive Index (26 C) 1.4852

No. 171

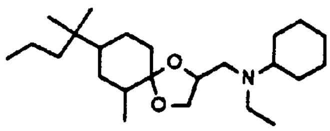

Refractive Index (26 C) 1.4852

No. 172

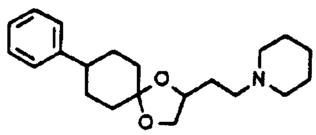

Refractive Index (26 C) 1.5275

No. 173

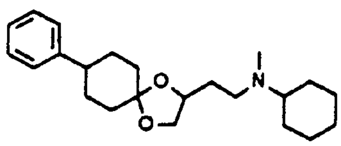

Refractive Index (26 C) 1.5272

No. 174

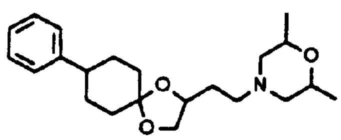

Refractive Index (26 C) 1.5188

No. 175

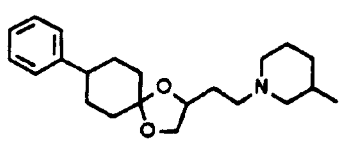

Refractive Index (26 C) 1.5203

No. 176

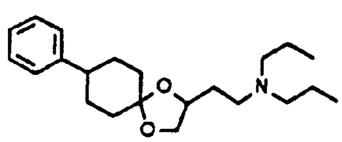

Refractive Index (26 C) 1.5118

No.
177
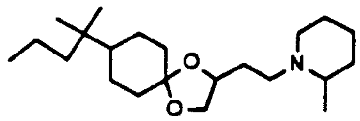
Refractive Index (26 C)
1.4842

---

No.
178
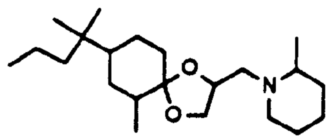
Refractive Index (26 C)
1.4840

---

No.
179
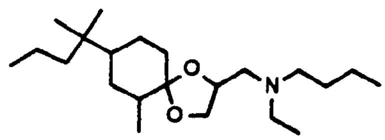
Refractive Index (26 C)
1.4693

---

No.
180
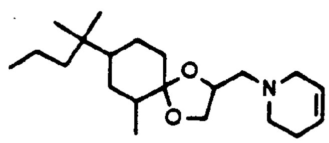
Refractive Index (26 C)
1.4926

---

No.
181
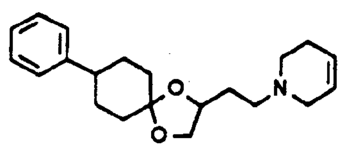
Refractive Index (26 C)
1.5354

---

No.
182
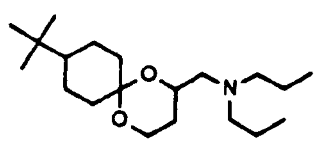
Refractive Index (26 C)
1.4680

---

No.
183
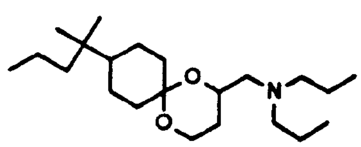
Refractive Index (26 C)
1.4715

---

No.
184
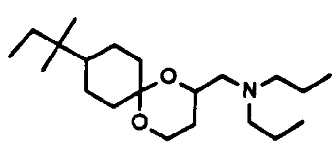
Refractive Index (26 C)
1.4699

---

No.

185

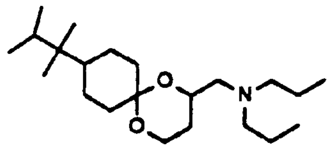

Refractive Index (26 C)

1.4735

---

No.

186

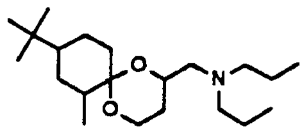

Refractive Index (26 C)

1.4666

---

No.

187

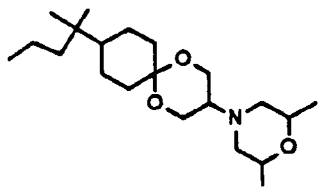

Refractive Index (26 C)

1.4872

---

No.

188

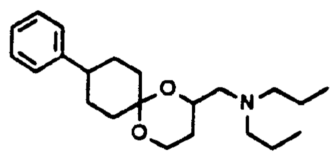

Refractive Index (26 C)

1.5044

---

No.

189

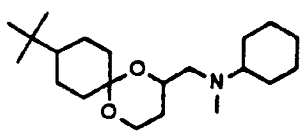

Bp 230°C/0.035mm Hg

---

No.

190

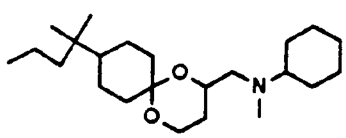

Refractive Index (26 C)

1.4909

---

No.

191

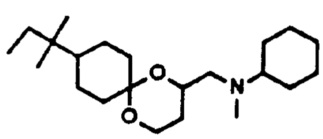

Refractive Index (26 C)

1.4913

---

No.

192

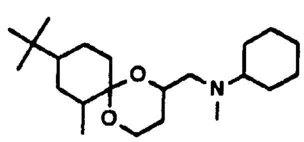

Refractive Index (26 C)

1.4877

---

No.

193

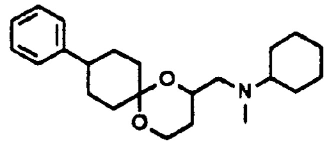

Refractive Index (26 C)

1.5291

---

No.

194

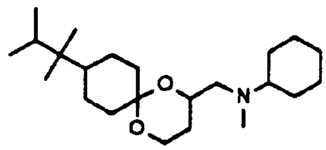

Refractive Index (26 C)

1.4947

---

No.

195

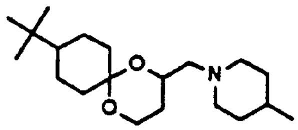

Refractive Index (26 C)

Bp 225°C/0.015mm Hg

---

No.

196

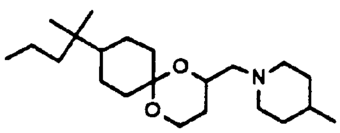

Refractive Index (26 C)

1.4859

---

No.

197

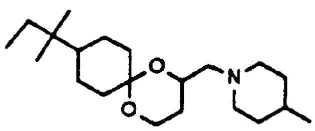

Refractive Index (26 C)

1.4851

---

No.

198

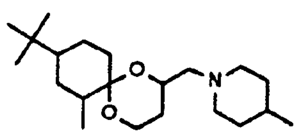

Refractive Index (26 C)

1.4812

---

No.

199

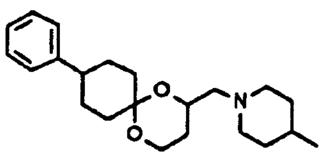

Refractive Index (26 C)

1.5237

---

No.

200

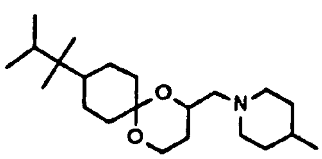

Refractive Index (26 C)

1.4894

---

No.
201

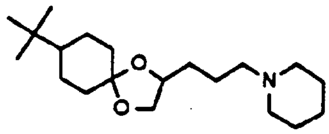

Refractive Index (26 C)
1.4815

---

No.
202

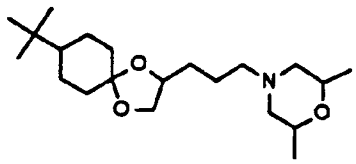

Refractive Index (26 C)
1.4765

---

No.
203

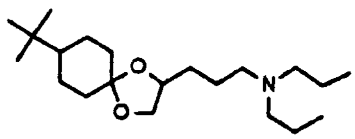

Refractive Index (26 C)
1.4658

---

No.
204

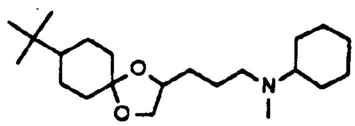

Refractive Index (26 C)
1.4839

---

No.
205

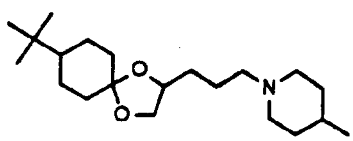

Refractive Index (26 C)
1.4784

---

No.
206

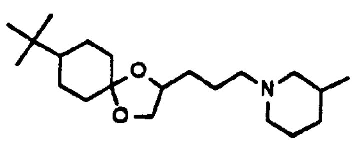

Refractive Index (26 C)
1.4792

---

No.
207

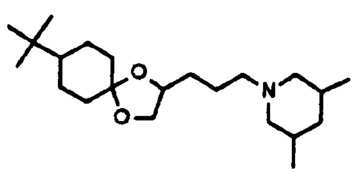

Refractive Index (26 C)
1.4774

---

No.
208

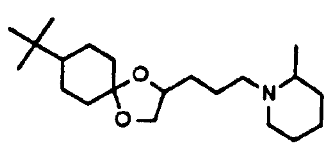

Refractive Index (26 C)
1.4815

---

No.

209

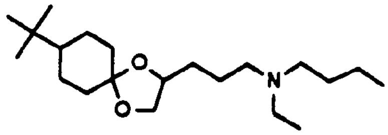

Refractive Index (26 C)

1.4667

No.

210

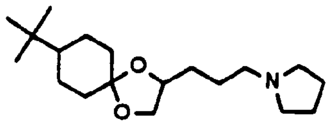

Refractive Index (26 C)

1.4802

No.

211

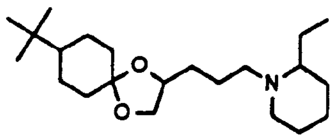

Refractive Index (26 C)

1.4826

No.

212

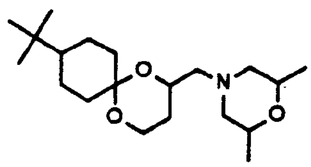

Refractive Index (26 C)

1.4806

No.

213

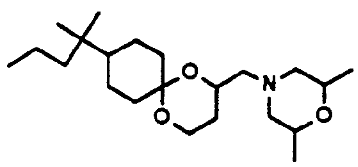

Refractive Index (26 C)

1.4841

No.

214

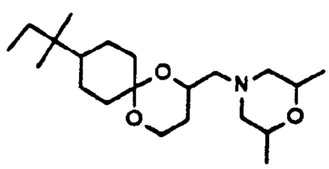

Refractive Index (26 C)

1.4841

No.

215

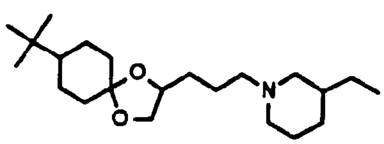

Refractive Index (26 C)

1.4800

No.

216

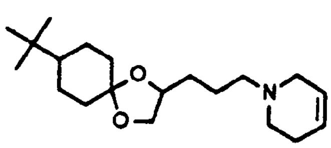

Refractive Index (26 C)

1.4886

No. 217

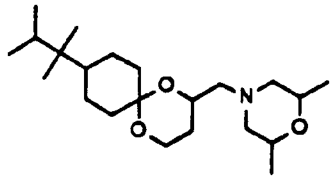

Refractive Index (26 C) 1.4886

---

No. 218

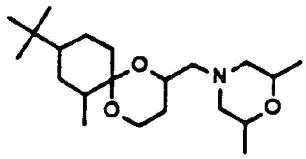

Refractive Index (26 C) 1.4796

---

No. 219

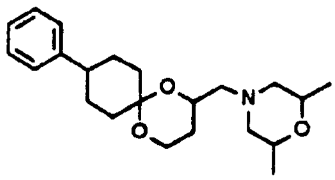

Refractive Index (26 C) 1.5222

---

No. 220

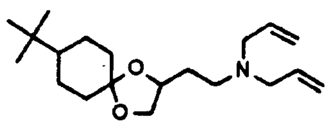

Refractive Index (26 C) 1.4784

---

No. 221

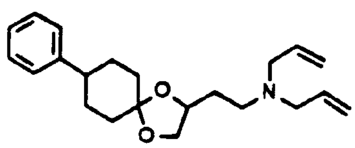

Refractive Index (26 C) 1.5204

---

No. 222

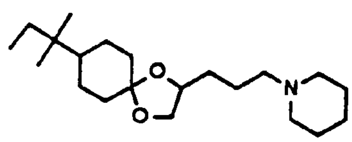

Refractive Index (26 C) 1.4842

---

No. 223

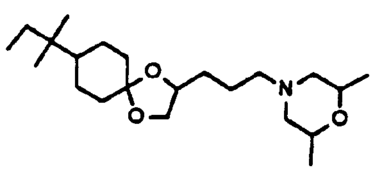

Refractive Index (26 C) 1.4794

---

No. 224

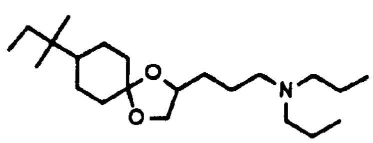

Refractive Index (26 C) 1.4692

---

No.

225

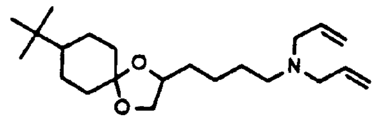

Refractive Index (26 C)
1.4781

---

No.

226

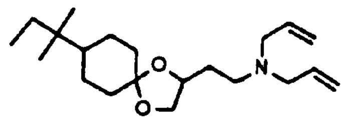

Refractive Index (26 C)
1.4812

---

No.

227

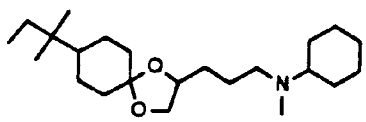

Refractive Index (26 C)
1.4861

---

No.

228

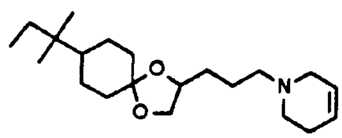

Refractive Index (26 C)
1.4920

---

No.

229

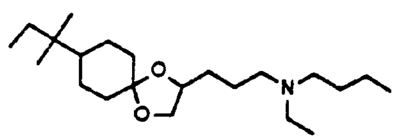

Refractive Index (26 C)
1.4993

---

No.

230

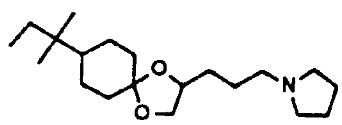

Refractive Index (26 C)
1.4830

---

No.

231

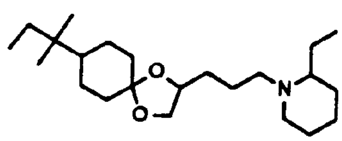

Refractive Index (26 C)
1.4849

---

No.

232

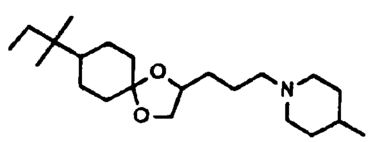

Refractive Index (26 C)
1.4810

---

No.

233

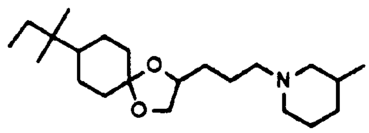

Refractive Index (26 C)
1.4819

No.

234

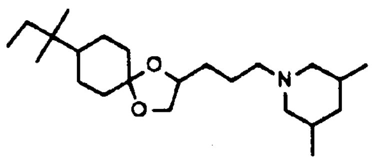

Refractive Index (26 C)
1.4806

No.

235

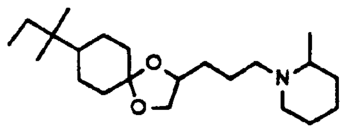

Refractive Index (26 C)
1.4842

No.

236

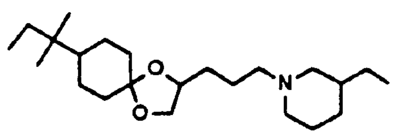

Refractive Index (26 C)
1.4824

No.

237

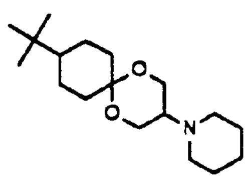

Mp 83 - 87°C

No.

238

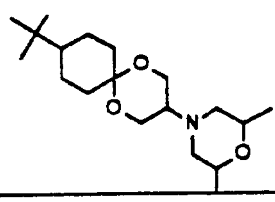

Refractive Index (26 C)
1.4862

No.

239

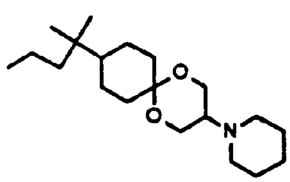

Refractive Index (26 C)
1.4946

No.

240

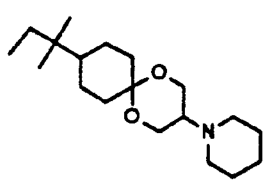

Mp 57 - 61°C

| | | |
|---|---|---|
| No. 241 | 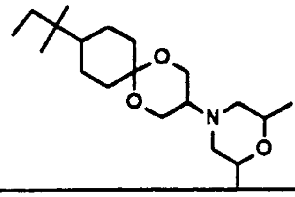 | Refractive Index (26 C) 1.4886 |
| No. 242 | 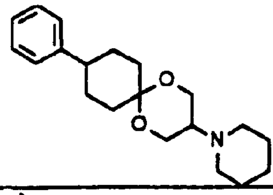 | Refractive Index (26 C) 1.5394 |
| No. 243 | 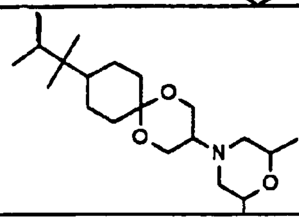 | Refractive Index (26 C) 1.4925 |
| No. 244 | 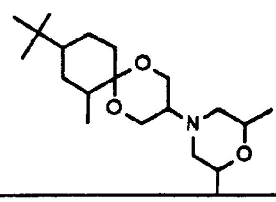 | Refractive Index (26 C) 1.4867 |
| No. 245 | 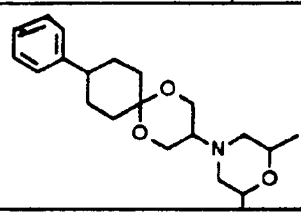 | Bp 235°C/0.0075mm Hg |

## APPLICATION EXAMPLES

### Example 1

Erysiphe graminis hordei (Mildew on barley).
Curative spraying of young barley plants.
Solvent : 10% acetone in water.
Emulsifier : Triton X-155, 100 ppm.

To prepare an appropriate composition of active substance the active substance is dissolved in the specified solvent with emulsifier to the concentration desired. Further dilution is carried out by a solution containing 10% acetone in water and 100 ppm Triton X-155.

To test for curative activity young barley plants are sprayed to leaf wetness with the composition of active substance 2 days after the plants have been inoculated with mildew conidia by the brush method. After inoculation and until spraying the plants are left at 18°C and 100% RH, and after spraying they are left in a growth chamber at 18°C and 70-80% RH.

7 Days after inoculation the activity of the substance is evaluated. In this test a clear superiority in activity is shown by, e.g., the compounds listed in Table 2.

The activity of the compounds is indicated as given below:

3 90-100% disease control
2 50- 89% disease control
1 11- 49% disease control

0 1- 10% disease control

**Example 2**

Puccinia recondita test (wheat, protective spray).
Solvent : 10% acetone in water.
Emulsifier : Triton® X-155, 100 ppm.

To prepare an appropriate composition of active substance the active substance is dissolved in the specified solvent with emulsifier to the concentration desired. Further dilution is carried out by a solution containing 10% acetone in water and 100 ppm Triton X-155.

To test for protective activity young wheat plants are sprayed to leaf wetness with the composition of active substance. After drying the plants are inoculated with brown rust spores in an aqueous slurry which is sprayed on. The plants are left for 48 hours at 18°C and 100% RH, whereafter they are transferred to a growth chamber at the same temperature and 70-80% RH.

10 Days after inoculation the activity of the substances is evaluated. In this test a clear superiority in activity is shown by, e.g., the compounds listed in Table 2.

The activity of the compounds is indicated as given below:

3 90-100% disease control
2 50- 89% disease control
1 11- 49% disease control
0 1- 10% disease control

**Example 3**

Botrytis cinerea test (cucumber leaf, protective test).
Solvent : 10% acetone in water
Emulsifier : Triton X-155, 100 ppm

To prepare an appropriate composition of active substance the active substance is dissolved in the specified solvent with emulsifier to the concentration desired. Further dilution is carried out by a solution containing 10% acetone in water and 100 ppm Triton X-155.

To test for protective activity cucumber leaf discs (diameter 30 mm) are soaked in the composition of the active compound for 1 min. After drying, small filter discs which have been immersed in a spore-hypha suspension of Botrytis cinerea are placed on the center of the cucumber leaf discs. The inoculated cucumber leaf discs are placed on glass plates in moist boxes.

After 2 - 3 days of incubation* at 23°C the activity of the active compound is evaluated (* when untreated control is overgrown with mycelium).

The activity of the active compounds is shown in Table 2 and is indicated as given below.

| | | |
|---|---|---|
| 3 complete | control | - no mycelial growth |
| 2 partial | control | - mycelial growth only on the filter disc |
| 1 partial | control | - inhibited mycelial growth on the cucumber leaf disc |
| 0 no | control | - uninhibited mycelial growth on the cucumber leaf disc |

**Example 4**

Erysiphe graminis tritici (Mildew on wheat).
Curative spraying of young wheat plants.

Solvent : 10% acetone in water.
Emulsifier : Triton® X-155, 100 ppm.

To prepare an appropriate composition of active substance the active substance is dissolved in the specified solvent with emulsifier to the concentration desired. Further dilution is carried out by a solution containing 10% acetone in water and 100 ppm Triton X-155.

To test for curative activity young wheat plants are sprayed to leaf wetness with the composition of active substance 24 hours after the plants have been inoculated with mildew conidia by the brush method. After inoculation and until spraying the plants are left at 18°C and 100% RH, and after spraying they are left in a growth chamber at 18°C and 70-80% RH.

8 Days after inoculation the activity of the substance is evaluated. In this test a clear superiority in activity is shown by, e.g., the compounds listed in Table 2.

The activity of the compounds is indicated as given below:

3 90-100% disease control
2 50- 89% disease control
1 11- 49% disease control
0 1- 10% disease control

Table 2

BIOLOGICAL TESTS

| Comp. No. | Erysiphe graminis hordei (barley) 64 ppm | Puccinia recondita (wheat) 256 ppm | Botrytis cinerea (cucumber) 256 ppm | Erysiphe graminis tritici (wheat) 64 ppm |
|---|---|---|---|---|
| 3 | 3 | 1 | 3 | |
| 4 | 3 | 2 | 3 | |
| 5 | 3 | 2 | 3 | |
| 9 | 3 | 2 | 2 | |
| 10 | 3 | 3 | 2 | |
| 19 | 3 | 3 | | |
| 33 | 3 | 3 | 3 | 3 |
| 35 | 3 | 3 | | 3 |
| 36 | 3 | 3 | | 3 |
| 38 | 3 | 2 | 2 | 3 |
| 40 | 3 | 2 | 2 | 3 |
| 41 | 3 | 3 | 3 | 3 |
| 42 | 3 | 3 | 3 | 3 |
| 43 | 2 | 1 | | 3 |
| 45 | 3 | 2 | | 3 |
| 46 | 3 | 3 | 3 | 3 |
| 47 | 3 | 3 | | |
| 48 | 3 | 2 | | |
| 49 | 3 | 3 | 3 | 3 |
| 50 | 3 | 2 | 1 | 3 |
| 51 | 3 | 3 | 3 | 3 |
| 54 | 3 | 3 | 1 | 3 |
| 55 | 3 | 2 | | |
| 56 | 3 | 3 | 2 | |
| 57 | 3 | 3 | | |
| 58 | 2 | 3 | 3 | |
| 62 | 3 | 3 | 2 | 3 |
| 63 | 3 | 3 | 3 | 3 |
| 64 | 3 | 3 | 1 | 3 |
| 65 | 3 | 3 | 3 | 3 |

## BIOLOGICAL TESTS

| Comp. No. | Erysiphe graminis hordei (barley) 64 ppm | Puccinia recondita (wheat) 256 ppm | Botrytis cinerea (cucumber) 256 ppm | Erysiphe graminis tritici (wheat) 64 ppm |
|---|---|---|---|---|
| 66 | 3 | 3 | 3 | 3 |
| 67 | 3 | 3 | 3 | 3 |
| 68 | 3 | 2 | | 3 |
| 69 | 3 | 3 | 2 | 3 |
| 70 | 3 | 3 | 2 | 3 |
| 71 | 3 | 3 | 2 | 3 |
| 72 | 3 | 3 | 3 | 3 |
| 73 | 3 | 3 | 3 | 3 |
| 74 | 2 | 3 | 2 | 3 |
| 75 | 2 | 3 | | |
| 76 | 3 | 3 | | 3 |
| 77 | 3 | 3 | | |
| 78 | 3 | 3 | 2 | 3 |
| 79 | 2 | 3 | | |
| 80 | 3 | 3 | 2 | 3 |
| 81 | 3 | 3 | 2 | 3 |
| 82 | 3 | 3 | 3 | 3 |
| 83 | 3 | 3 | 3 | 3 |
| 84 | 3 | 3 | 3 | 3 |
| 85 | 3 | | | |
| 87 | 3 | 3 | 3 | 3 |
| 88 | 3 | 3 | 3 | 3 |
| 91 | 3 | 3 | | 3 |
| 92 | 3 | 3 | 2 | 3 |
| 93 | 3 | 3 | 3 | 3 |
| 94 | 3 | 3 | 3 | |
| 95 | 3 | 3 | 3 | 3 |
| 96 | 3 | 3 | 3 | 3 |
| 97 | 3 | 3 | 3 | 3 |
| 98 | 3 | 3 | 3 | 3 |

BIOLOGICAL TESTS

| Comp. No. | Erysiphe graminis hordei (barley) 64 ppm | Puccinia recondita (wheat) 256 ppm | Botrytis cinerea (cucumber) 256 ppm | Erysiphe graminis tritici (wheat) 64 ppm |
|---|---|---|---|---|
| 99 | 3 | 3 | 3 | 3 |
| 100 | 3 | 3 | 2 | |
| 101 | 3 | 2 | 2 | 3 |
| 102 | 3 | 3 | 3 | 3 |
| 103 | 3 | 3 | 3 | 3 |
| 104 | 3 | 3 | 2 | 3 |
| 105 | 3 | 3 | 3 | 3 |
| 106 | 3 | 3 | | |
| 107 | 3 | 3 | 2 | 3 |
| 108 | 3 | 3 | 3 | 3 |
| 109 | 3 | 3 | 2 | 3 |
| 110 | 3 | 3 | 2 | 3 |
| 111 | 3 | 3 | 2 | 3 |
| 112 | 3 | 3 | 2 | 3 |
| 115 | 3 | 3 | 2 | 3 |
| 116 | 3 | 3 | 3 | 3 |
| 128 | 3 | 3 | 2 | 3 |
| 129 | 3 | 3 | 2 | 3 |
| 131 | 3 | 3 | | 3 |
| 137 | 2 | 3 | | |
| 143 | 3 | 3 | | |
| 145 | 3 | 3 | 2 | 3 |
| 146 | 3 | 3 | | 3 |
| 147 | 3 | 3 | 3 | 3 |
| 148 | 3 | 3 | 3 | 3 |
| 149 | 3 | 3 | 2 | 3 |
| 151 | 3 | 3 | 2 | 3 |
| 152 | 3 | 3 | 2 | 3 |
| 153 | 3 | 3 | 2 | |
| 154 | 3 | 3 | 3 | 3 |

BIOLOGICAL TESTS

| Comp. No. | Erysiphe graminis hordei (barley) 64 ppm | Puccinia recondita (wheat) 256 ppm | Botrytis cinerea (cucumber) 256 ppm | Erysiphe graminis tritici (wheat) 64 ppm |
|---|---|---|---|---|
| 155 | 3 | 3 | 3 | 3 |
| 156 | 2 | 3 | 2 | |
| 158 | | 3 | | |
| 159 | | 3 | 3 | 3 |
| 160 | | 3 | 2 | |
| 161 | 3 | 3 | 3 | 3 |
| 165 | 3 | 3 | | |
| 166 | 3 | 2 | | 3 |
| 171 | | 2 | | 3 |
| 177 | | 3 | 2 | 2 |
| 178 | | 3 | 2 | 3 |
| 220 | | | 3 | 3 |
| 226 | | | 3 | 3 |

## Claims

**Claims for the following Contracting States : AT, BE, CH, DE, FR, GB, IT, LI, LU, NL, SE, GR**

**1.** Amino ketals , characterised by having the general formula I

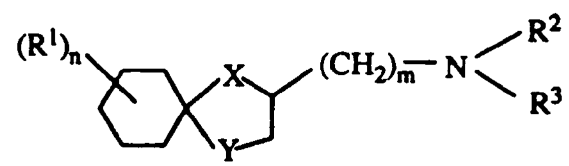

I

wherein X and Y each independently represents O or $-OCH_2-$,
$(R^1)_n$ represents up to four substituents which are the same or different and selected from straight or branched chain alkyl having up to 10 carbon atoms, cycloalkyl having up to 6 carbon atoms, aryl, aryloxy, alkoxy, cycloalkoxy, arylalkyl and alkylthio, where two adjacent $R^1$-substituents may also together form a saturated or unsaturated ring, and
n is 1, 2, 3 or 4, and m is 0, 1, 2, 3 or 4, and
$R^2$ and $R^3$ each independently represents hydrogen, straight or branched chain alkyl having up to 10 carbon atoms, unsubstituted or alkylsubstituted cycloalkyl, alkenyl, alkynyl or unsubstituted or alkylsubstituted arylalkyl, or
$R^2$ and $R^3$ when taken together with the nitrogen atom to which they are attached form a saturated or unsaturated, substituted or unsubstituted heterocyclic ring which may optionally be aryl-fused or cycloalk-

ylfused and which may optionally contain one or more additional hetero atoms selected from O and N, with the proviso that if X and Y are both O, m is 1, and $-NR^2R^3$ is other than an unsaturated, substituted or unsubstituted heterocyclic ring which may optionally be aryl-fused or cycloalkyl-fused and which may optionally contain one or more additional hetero atoms selected from O and N, then $(R^1)_n$ cannot be a sole substituent (n=1) in the 4-position in the cyclohexane ring and of the formula

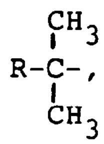

wherein R represents hydrogen, alkyl or optionally substituted phenyl, and with the further proviso that if X and Y are both O, m is 1, and $-NR^2R^3$ is amino or phthalimido, then $(R^1)_n$ cannot be solely 8-methyl, 8-methoxy or 6-phenyl or solely 7,7,9,9-tetramethyl.

**2.** A process for the preparation of amino ketals of the general formula I

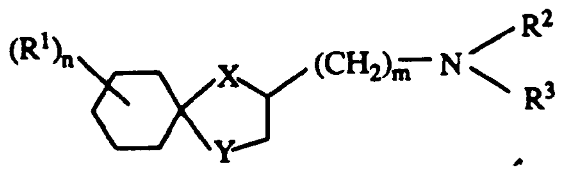

I

wherein X, Y, $R^1$, $R^2$, $R^3$, n and m have the meanings defined in claim 1, <u>characterised</u> by reacting a ketal of the general formula II

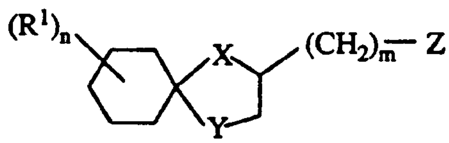

II

wherein X, Y, $(R^1)_n$ and m have the above defined meanings, and wherein Z represents an exchangeable electron-attracting group, with an amine of the general formula III

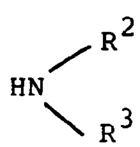

III

wherein $R^2$ and $R^3$ have the above defined meanings, optionally in the presence of a diluent and optionally in the presence of a catalyst, and optionally in the presence of an acid-binding agent in addition to a possible excess of the amine III.

**3.** A fungicidal composition, particularly a plant-fungicidal composition, and more particularly a cereal-fungicidal composition for combating mildew, rust and other significant foliage diseases, <u>characterised</u> by containing as an active component at least one compound selected from the amino ketals of formula I as defined in claim 1.

**4.** Use of an amino ketal of formula I as defined in claim 1 for combating fungi, particularly phytopathogenic fungi, and more particularly mildew, rust and other significant foliage diseases on cereals.

**5.** A method of combating fungi, particularly phytopathogenic fungi, and more particularly mildew, rust and

other significant foliage diseases on cereals, characterised by allowing an amino ketal of formula I as defined in claim 1 to affect the fungi concerned and/or their biotope.

6. A process of preparing a fungicidal composition, particularly a plant-fungicidal composition, and more particularly a cereal-fungicidal composition for combating mildew, rust and other significant foliage diseases, characterised by mixing an amino ketal of formula I as defined in claim 1 with one or more agents selected from extending agents, surface active agents and other conventional auxiliary agents.

**Claims for the following Contracting State : ES**

1. A process for the preparation of amino ketals of the genral formula I

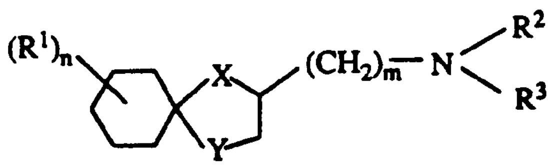

I

wherein X and Y each independently represents O or $-OCH_2-$,
$(R^1)_n$ represents up to four substituents which are the same or different and selected from straight or branched chain alkyl having up to 10 carbon atoms, cycloalkyl having up to 6 carbon atoms, aryl, aryloxy, alkoxy, cycloalkoxy, arylalkyl and alkylthio, where two adjacent $R^1$-substituents may also together form a saturated or unsaturated ring, and
n is 1, 2, 3 or 4, and m is 0, 1, 2, 3 or 4, and
$R^2$ and $R^3$ each independently represents hydrogen, straight or branched chain alkyl having up to 10 carbon atoms, unsubstituted or alkylsubstituted cycloalkyl, alkenyl, alkynyl or unsubstituted or alkylsubstituted arylalkyl, or
$R^2$ and $R^3$ when taken together with the nitrogen atom to which they are attached form a saturated or unsaturated, substituted or unsubstituted heterocyclic ring which may optionally be aryl-fused or cycloalkylfused and which may optionally contain one or more additional hetero atoms selected from O and N, with the proviso that if X and Y are both O, m is 1, and $-NR^2R^3$ is other than an unsaturated, substituted or unsubstituted heterocyclic ring which may optionally be aryl-fused or cycloalkyl-fused and which may optionally contain one or more additional hetero atoms selected from O and N, then $(R^1)_n$ cannot be a sole substituent (n=1) in the 4-position in the cyclohexane ring and of the formula

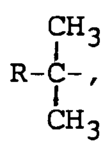

wherein R represents hydrogen, alkyl or optionally substituted phenyl, and with the further proviso that if X and Y are both O, m is 1, and $-NR^2R^3$ is amino or phthalimido, then $(R^1)_n$ cannot be solely 8-methyl, 8-methoxy or 6-phenyl or solely 7,7,9,9-tetramethyl, characterised by reacting a ketal of the general formula II

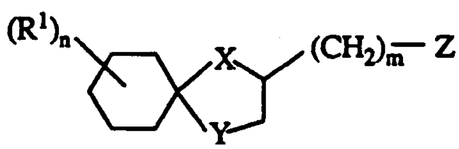

II

wherein X, Y, $(R^1)_n$ and m have the above defined meanings, and wherein Z represents an exchangeable electron-attracting group, with an amine of the general formula III

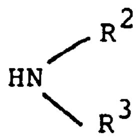

III

wherein $R^2$ and $R^3$ have the above defined meanings, optionally in the presence of a diluent and optionally in the presence of a catalyst, and optionally in the presence of an acid-binding agent in addition to a possible excess of the amine III.

2. A fungicidal composition, particularly a plant-fungicidal composition, and more particularly a cereal-fungicidal composition for combating mildew, rust and other significant foliage diseases, characterised by containing as an active component at least one compound selected from the amino ketals of the general formula I

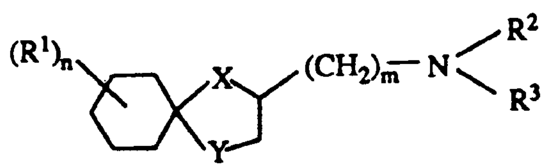

I

wherein X and Y each independently represents O or $-OCH_2-$,
$(R^1)_n$ represents up to four substituents which are the same or different and selected from straight or branched chain alkyl having up to 10 carbon atoms, cycloalkyl having up to 6 carbon atoms, aryl, aryloxy, alkoxy, cycloalkoxy, arylalkyl and alkylthio, where two adjacent $R^1$-substituents may also together form a saturated or unsaturated ring, and
n is 1, 2, 3 or 4, and m is 0, 1, 2, 3 or 4, and
$R^2$ and $R^3$ each independently represents hydrogen, straight or branched chain alkyl having up to 10 carbon atoms, unsubstituted or alkylsubstituted cycloalkyl, alkenyl, alkynyl or unsubstituted or alkylsubstituted arylalkyl, or
$R^2$ and $R^3$ when taken together with the nitrogen atom to which they are attached form a saturated or unsaturated, substituted or unsubstituted heterocyclic ring which may optionally be aryl-fused or cycloalkylfused and which may optionally contain one or more additional hetero atoms selected from O and N, with the proviso that if X and Y are both O, m is 1, and $-NR^2R^3$ is other than an unsaturated, substituted or unsubstituted heterocyclic ring which may optionally be aryl-fused or cycloalkyl-fused and which may optionally contain one or more additional hetero atoms selected from O and N, then $(R^1)_n$ cannot be a sole substituent (n=1) in the 4-position in the cyclohexane ring and of the formula

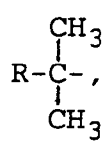

wherein R represents hydrogen, alkyl or optionally substituted phenyl, and with the further proviso that if X and Y are both O, m is 1, and $-NR^2R^3$ is amino or phthalimido, then $(R^1)_n$ cannot be solely 8-methyl, 8-methoxy or 6-phenyl or solely 7,7,9,9-tetramethyl.

3. Use of an amino ketal of formula I as defined in claim 2 for combating fungi, particularly phytopathogenic fungi, and more particularly mildew, rust and other significant foliage diseases on cereals.

4. A method of combating fungi, particularly phytopathogenic fungi, and more particularly mildew, rust and other significant foliage diseases on cereals, characterised by allowing an amino ketal of formula I as defined in claim 2 to affect the fungi concerned and/or their biotope.

5. A process of preparing a fungicidal composition, particularly a plant-fungicidal composition, and more particularly a cereal-fungicidal composition for combating mildew, rust and other significant foliage diseases, characterised by mixing an amino ketal of formula I as defined in claim 2 with one or more agents selected from extending agents, surface active agents and other conventional auxiliary agents.

## Patentansprüche

### Patentansprüche für folgende Vertragsstaaten : AT, BE, CH, DE, FR, GB, IT, LI, LU, NL, SE, GR

1. Aminoketale, dadurch **gekennzeichnet,** dass sie die allgemeine Formel I

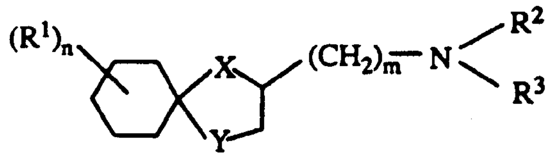

I

aufweisen,
in der X und Y unabhängig voneinander jeweils O oder $-OCH_2-$ bedeutet,
$(R^1)_n$ bis zu vier gleiche oder verschiedene Substituenten, ausgewählt aus geradkettigem oder verzweigtem Alkyl mit bis zu 10 Kohlenstoffatomen, Cycloalkyl mit bis zu 6 Kohlenstoffatomen, Aryl, Aryloxy, Alkoxy, Cycloalkoxy, Arylalkyl und Alkylthio bedeutet, wobei zwei benachbarte $R^1$-Substituenten auch zusammen einen gesättigten oder ungesättigten Ring bilden können,
n 1, 2, 3 oder 4 ist und m 0, 1, 2, 3 oder 4 ist, und
$R^2$ und $R^3$ unabhängig voneinander jeweils Wasserstoff, geradkettiges oder verzweigtes Alkyl mit bis zu 10 Kohlenstoffatomen, unsubstituiertes oder alkylsubstiiertes Cycloalkyl, Alkenyl, Alkinyl oder unsubstituiertes oder alkylsubstituiertes Arylalkyl bedeutet, oder
$R^2$ und $R^3$ gemeinsam mit dem Stickstoffatom, an welches sie gebunden sind, einen gesättigten oder ungesättigten, substituierten oder unsubstituierten heteroryclischen Ring bilden, der gegebenenfalls arylkondensiert oder cycloalkylkondensiert sein kann und gegebenenfalls ein oder mehrere unter O und N ausgewählte zusätzliche Heteroatome enthalten kann,
mit dem Vorbehalt, dass, wenn X und Y beide O sind, m 1 ist, und $-NR^2R^3$ anderes als ein ungesättigter, substituierter oder unsubstituierter heterocyclischer Ring ist, der gegebenenfalls arylkondensiert oder cycloalkylkondensiert sein kann, und gegebenenfalls ein oder mehrere unter O und N ausgewählte zusätzliche Heteroatome enthalten kann, $(R^1)_n$ dann nicht ein einzelner Substituent (n=1) in der 4-Stellung im Cyclohexanring und mit der Formel

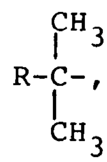

in der R Wasserstoff, Alkyl oder gegebenenfalls substituiertes Phenyl bedeutet, sein kann,
und mit dem weiteren Vorbehalt, dass, wenn X und Y beide O sind, m 1 ist, und $-NR^2R^3$ Amino oder Phthalimido ist, $(R^1)_n$ dann nicht allein 8-Methyl, 8-Methoxy oder 6-Phenyl oder allein 7,7,9,9-Tetramethyl sein kann.

2. Verfahren zur Herstellung von Aminoketalen der allgemeinen Formel I

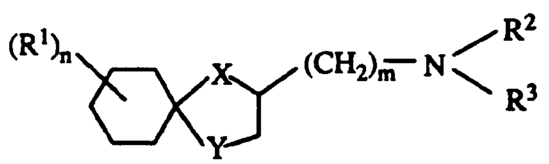

I

in der X, Y, $R^1$, $R^2$, $R^3$, n und m die in Anspruch 1 angeführten Bedeutungen haben, dadurch **gekennzeichnet,** dass ein Ketal der allgemeinen Formel II

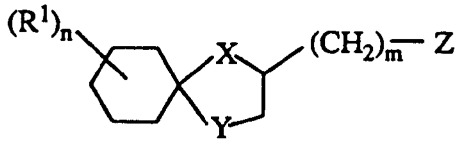

II

in der X, Y, $(R^1)_n$ und m die oben angeführten Bedeutungen haben, und in der Z eine auswechselbare elektronenanziehende Gruppe bedeutet, mit einem Amin der allgemeinen Formel III

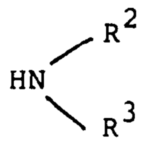

III

in der $R^2$ und $R^3$ die oben angeführten Bedeutungen haben, gegebenenfalls in Gegenwart eines Verdünnungsmittels und gegebenenfalls in Gegenwart eines Katalysators, und gegebenenfalls in Gegenwart eines säurebindenden Mittels ausser einem eventuellen Überschuss des Amins III umgesetzt wird.

3. Fungizides Mittel, insbesondere pflanzen-fungizides Mittel, und insbesondere getreide-fungizides Mittel zur Bekämpfung von Mehltau, Rost und anderen bedeutenden Blattktrankheiten, dadurch **gekennzeichnet,** dass es als aktiver Bestandteil zumindest eine aus den Aminoketalen der Formel I in Anspruch 1 ausgewählte Verbindung enthält.

4. Verwendung eines Aminoketals der Formel I nach Anspruch 1 zur Bekämpfung von Pilzen, namentlich pflanzenpathogenen Pilzen, und insbesondere Mehltau, Rost und anderen bedeutenden Blattkrankheiten des Getreides.

5. Verfahren zur Bekämpfung von Pilzen, namentlich pflanzenpathogenen Pilzen, und insbesondere Mehltau, Rost und anderen bedeutenden Blattkrankheiten des Getreides, dadurch **gekennzeichnet,** dass man ein Aminoketal der Formel I nach Anspruch 1 auf den betreffenden Pilze und/oder deren Biotop einwirken lässt.

6. Verfahren zur Herstellung eines fungiziden Mittels, namentlich eines pflanzen-fungiziden Mittels, und insbesondere eines getreide-fungiziden Mittels zur Bekämpfung von Mehltau, Rost und anderen bedeutenden Blattkrankheiten, dadurch **gekennzeichnet,** dass man ein Aminoketal der Formel I nach Anspruch 1 mit einem oder mehreren unter Streckmitteln, oberflächenaktiven Mitteln und anderen herkömmlichen Hilfsmitteln ausgewählten Mitteln mischt.

**Patentansprüche für folgenden Vertragsstaat : ES**

1. Verfahren zur Herstellung von Aminoketalen der allgemeinen Formel I

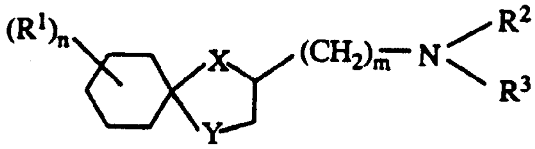

I

in der X und Y unabhängig voneinander jeweils O oder $-OCH_2-$ bedeutet,

$(R^1)_n$ bis zu vier gleiche oder verschiedene Substituenten, ausgewählt aus geradkettigem oder verzweigtem Alkyl mit bis zu 10 Kohlenstoffatomen, Cycloalkyl mit bis zu 6 Kohlenstoffatomen, Aryl, Aryloxy, Alkoxy, Cycloalkoxy, Arylalkyl und Alkylthio bedeutet, wobei zwei benachbarte $R^1$-Substituenten auch zusammen einen gesättigten oder ungesättigten Ring bilden können,
n 1, 2, 3 oder 4 ist und m 0, 1, 2, 3 oder 4 ist, und
$R^2$ und $R^3$ unabhängig voneinander jeweils Wasserstoff, geradkettiges oder verzweigtes Alkyl mit bis zu 10 Kohlenstoffatomen, unsubstituiertes oder alkylsubstiiertes Cycloalkyl, Alkenyl, Alkinyl oder unsubstituiertes oder alkylsubstituiertes Arylalkyl bedeutet, oder
$R^2$ und $R^3$ gemeinsam mit dem Stickstoffatom, an welches sie gebunden sind, einen gesättigten oder ungesättigten, substituierten oder unsubstituierten heterocyclischen Ring bilden, der gegebenenfalls arylkondensiert oder cycloalkylkondensiert sein kann und gegebenenfalls ein oder mehrere unter O und N ausgewählte zusätzliche Heteroatome enthalten kann,
mit dem Vorbehalt, dass, wenn X und Y beide O sind, m 1 ist, und $-NR^2R^3$ anderes als ein ungesättigter, substituierter oder unsubstituierter heterocyclischer Ring ist, der gegebenenfalls arylkondensiert oder cycloalkylkondensiert sein kann, und gegebenenfalls ein oder mehrere unter O und N ausgewählte zusätzliche Heteroatome enthalten kann, $(R^1)_n$ dann nicht ein einzelner Substituent (n=1) in der 4-Stellung im Cyclohexanring und mit der Formel

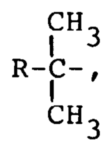

in der R Wasserstoff, Alkyl oder gegebenenfalls substituiertes Phenyl bedeutet, sein kann,
und mit dem weiteren Vorbehalt, dass, wenn X und Y beide O sind, m 1 ist, und $-NR^2R^3$ Amino oder Phthalimido ist, $(R^1)_n$ dann nicht allein 8-Methyl, 8-Methoxy oder 6-Phenyl oder allein 7,7,9,9-Tetramethyl sein kann, dadurch **gekennzeichnet,** dass ein Ketal der allgemeinen Formel II

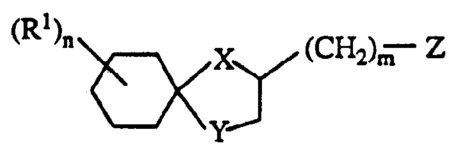

II

in der X, Y, $(R^1)_n$ und m die oben angeführten Bedeutungen haben, und in der Z eine auswechselbare elektronenanziehende Gruppe bedeutet, mit einem Amin der allgemeinen Formel III

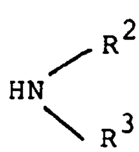

III

in der $R^2$ und $R^3$ die oben angeführten Bedeutungen haben, gegebenenfalls in Gegenwart eines Verdünnungsmittels und gegebenenfalls in Gegenwart eines Katalysators, und gegebenenfalls in Gegenwart eines säurebindenden Mittels ausser einem eventuellen Überschuss des Amins III umgesetzt wird.

**2.** Fungizides Mittel, namentlich pflanzen-fungizides Mittel, und insbesondere getreide-fungizides Mittel zur Bekämpfung von Mehltau, Rost und anderen bedeutenden Blattkrankheiten, dadurch **gekennzeichnet,** dass es als aktiver Bestandteil zumindest eine aus den Aminoketalen der Formel I

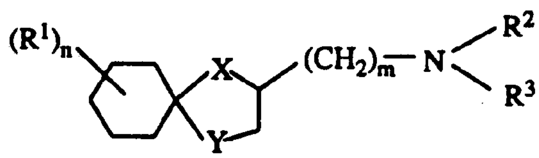

I

ausgewählte Verbindung enthält,
in der X und Y unabhängig voneinander jeweils O oder $-OCH_2-$ bedeutet,
$(R^1)_n$ bis zu vier gleiche oder verschiedene Substituenten, ausgewählt aus geradkettigem oder verzweigtem Alkyl mit bis zu 10 Kohlenstoffatomen, Cycloalkyl mit bis zu 6 Kohlenstoffatomen, Aryl, Aryloxy, Alkoxy, Cycloalkoxy, Arylalkyl und Alkylthio bedeutet, wobei zwei benachbarte $R^1$-Substituenten auch zusammen einen gesättigten oder ungesättigten Ring bilden können,
n 1, 2, 3 oder 4 ist und m 0, 1, 2, 3 oder 4 ist, und
$R^2$ und $R^3$ unabhängig voneinander jeweils Wasserstoff, geradkettiges oder verzweigtes Alkyl mit bis zu 10 Kohlenstoffatomen, unsubstituiertes oder alkylsubstiiertes Cycloalkyl, Alkenyl, Alkinyl oder unsubstituiertes oder alkylsubstituiertes Arylalkyl bedeutet, oder
$R^2$ und $R^3$ gemeinsam mit dem Stickstoffatom, an welches sie gebunden sind, einen gesättigten oder ungesättigten, substituierten oder unsubstituierten heterocyclischen Ring bilden, der gegebenenfalls arylkondensiert oder cycloalkylkondensiert sein kann und gegebenenfalls ein oder mehrere unter O und N ausgewählte zusätzliche Heteroatome enthalten kann,
mit dem Vorbehalt, dass, wenn X und Y beide O sind, m 1 ist, und $-NR^2R^3$ anderes als ein ungesättigter, substituierter oder unsubstituierter heterocyclischer Ring ist, der gegebenenfalls arylkondensiert oder cycloalkylkondensiert sein kann, und gegebenenfalls ein oder mehrere unter O und N ausgewählte zusätzliche Heteroatome enthalten kann, $(R^1)_n$ dann nicht ein einzelner Substituent (n=1) in der 4-Stellung im Cyclohexanring und mit der Formel

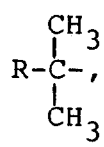

in der R Wasserstoff, Alkyl oder gegebenenfalls substituiertes Phenyl bedeutet, sein kann,
und mit dem weiteren Vorbehalt, dass, wenn X und Y beide O sind, m 1 ist, und $-NR^2R^3$ Amino oder Phthalimido ist, $(R^1)_n$ dann nicht allein 8-Methyl, 8-Methoxy oder 6-Phenyl oder allein 7,7,9,9-Tetramethyl sein kann.

3. Verwendung eines Aminoketals der Formel I nach Anspruch 2 zur Bekämpfung von Pilzen, namentlich pflanzenpathogenen Pilzen, und insbesondere Mehltau, Rost und anderen bedeutenden Blattkrankheiten des Getreides.

4. Verfahren zur Bekämpfung von Pilzen, namentlich pflanzenpathogenen Pilzen, und insbesondere Mehltau, Rost und anderen bedeutenden Blattkrankheiten des Getreides, dadurch **gekennzeichnet,** dass man ein Aminoketal der Formel I nach Anspruch 2 auf die betreffenden Pilze und/oder deren Biotop einwirken lässt.

5. Verfahren zur Herstellung eines fungiziden Mittels, namentlich eines pflanzen-fungiziden Mittels, und insbesondere eines getreide-fungiziden Mittels zur Bekämpfung von Mehltau, Rost und anderen bedeutenden Blattkrankheiten, dadurch **gekennzeichnet,** dass man ein Aminoketal der Formel I nach Anspruch 2 mit einem oder mehreren unter Streckmitteln, oberflächenaktiven Mitteln und anderen herkömmlichen Hilfsmitteln ausgewählten Mitteln mischt.

## Revendications

**Revendications pour les Etats contractants suivants : AT, BE, CH, DE, FR, GB, IT, LI, LU, NL, SE, GR**

1. Aminocétals, caractérisé en ce qu'ils répondent à la formule générale I

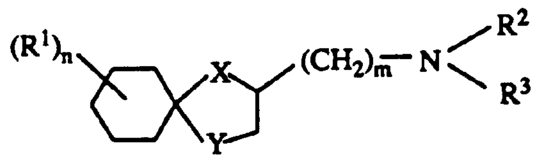

I

dans laquelle X et Y représentent chacun, indépendamment l'un de l'autre, O ou $-OCH_2-$, $(R^1)_n$ représente jusqu'à quatre substituants identiques ou différents choisis parmi des groupes alkyle à chaîne droite ou ramifiée et contenant jusqu'à 10 atomes de carbone, cycloalkyle contenant jusqu'à 6 atomes de carbone, aryle, aryloxy, alcoxy, cycloalcoxy, arylalkyle et alkythio, deux substituants $R^1$ voisins pouvant aussi former conjointement un anneau saturé ou non saturé, et
n est 1, 2, 3 ou 4, et m est 0, 1, 2, 3 ou 4, et
$R^2$ et $R^3$ représentent chacun, indépendamment l'un de l'autre, un atome d'hydrogène, un groupe alkyle à chaîne droite ou ramifiée et contenant jusqu'à 10 atomes de carbone, un groupe cycloalkyle non substitué ou substitué par alkyle, un groupe alcényle, un groupe alcynyle ou un groupe arylalkyle non substitué ou substitué par alkyle, ou
$R^2$ et $R^3$ représentent, conjointement avec l'atome d'azote auquel ils sont liés, un hétérocycle saturé ou non saturé, substitué ou non substitué, qui peut être éventuellement aryle-condensé ou cycloalkyle-condensé et qui peut contenir éventuellement un ou plusieurs hétéroatomes supplémentaires choisis parmi O et N,
sous la réserve que si X et Y sont tous les deux O, m est 1 et $-NR^2R^3$ est autre qu'un hétérocycle non saturé, substitué ou non substitué, qui peut être éventuellement aryle-condensé ou cycloalkyle-condensé et qui peut contenir éventuellement un ou plusieurs hétéroatomes supplémentaires choisis parmi O et N, $(R^1)_n$ ne peut alors pas être un substituant unique (n = 1) en position 4 de l'anneau cyclohexane et ayant la formule

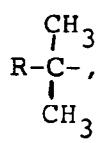

dans laquelle R représente un atome d'hydrogène, un groupe alkyle ou un groupe phényle éventuellement substitué,
et sous la réserve supplémentaire que si X et Y sont tous les deux O, m est 1 et $-NR^2R^3$ est un groupe amino ou phtalimido, $(R^1)_n$ ne peut alors pas être seulement 8-méthyle, 8-méthoxy ou 6-phényle ou seulement 7,7,9,9-tetraméthyle.

2. Procédé pour la préparation d'aminocétals répondant à la formule générale I

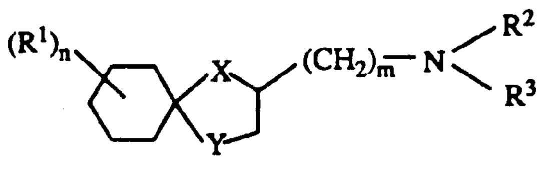

I

dans laquelle X, Y, $R^1$, $R^2$, $R^3$, n et m ont les significations définies dans la revendication 1, caractérisé en ce qu'on fait réagir un cétal de formule générale II

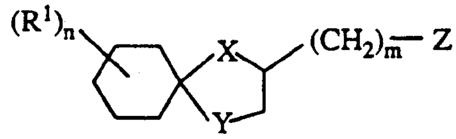

II

dans laquelle X, Y, $(R^1)_n$ et m ont les significations définies ci-dessus, et dans laquelle Z représente une groupe échangeable attirant les électrons, avec une amine de formule générale III

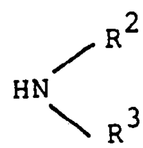

III

dans laquelle $R^2$ et $R^3$ ont les significations définies ci-dessus, éventuellement en présence d'un diluant et éventuellement en présence d'un catalyseur, et éventuellement en présence d'un fixateur d'acide en surplus d'un excédent éventuel de l'amine III.

3. Agent fongicide, particulièrement agent fongicide sur plantes, et plus particulièrement agent fongicide sur céréales, pour la lutte contre le mildiou, la rouille et autres maladies significatives de feuillages, caractérisé en ce qu'il contient, comme composant actif, au moins un composé choisi parmi les aminocétals de formule I selon la revendication 1.

4. Utilisation d'un aminocétal de formule I selon la revendication 1 pour la lutte contre champignons, particulièrement champignons phytopathogènes, et plus particulièrement le mildiou, la rouille et autres maladies significatives des feuillages de céréales.

5. Procédé pour la lutte contre des champignons, particulièrement champignons phytopathogènes, et plus particulièrement le mildiou, la rouille et autres maladies significatives des feuillages de céréales, caractérisé en ce qu'on laisse agir un aminocétal de formule I selon la revendication 1 sur les champignons concernés et/ou dans leur biotope.

6. Procédé pour la préparation d'un agent fongicide, particulièrement d'un agent fongicide sur plantes, et plus particulièrement d'un agent fongicide sur céréales, pour la lutte contre le mildiou, la rouille et autres maladies significatives des feuillages, caractérisé en ce qu'on mélange un aminocétal de formule I selon la revendication 1 avec un ou plusieurs agents choisis parmi des charges, des agents tensioactifs et d'autres agents auxiliaires usuels.

**Revendications pour l'Etat contractant suivant : ES**

1. Procédé pour la préparation d'aminocétals répondant à la formule générale I

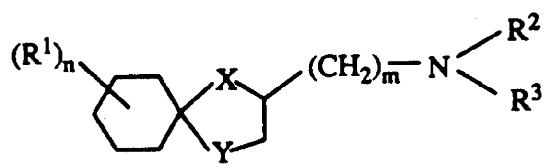

I

dans laquelle X et Y représentent chacun, indépendamment l'un de l'autre, O ou $-OCH_2-$,
$(R^1)_n$ représente jusqu'à quatre substituants identiques ou différents choisis parmi des groupes alkyle à chaîne droite ou ramifiée et contenant jusqu'à 10 atomes de carbone, cycloalkyle contenant jusqu'à 6 atomes de carbone, aryle, aryloxy, alcoxy, cycloalcoxy, arylalkyle et alkythio, deux substituants $R^1$ voisins pouvant aussi former conjointement un anneau saturé ou non saturé, et
n est 1, 2, 3 ou 4, et m est 0, 1, 2, 3 ou 4, et
$R^2$ et $R^3$ représentent chacun, indépendamment l'un de l'autre, un atome d'hydrogène, un groupe alkyle

à chaîne droite ou ramifiée et contenant jusqu'à 10 atomes de carbone, un groupe cycloalkyle non substitué ou substitué par alkyle, un groupe alcényle, un groupe alcynyle ou un groupe arylalkyle non substitué ou substitué par alkyle, ou

$R^2$ et $R^3$ représentent, conjointement avec l'atome d'azote auquel ils sont liés, un hétérocycle saturé ou non saturé, substitué ou non substitué, qui peut être éventuellement aryle-condensé ou cycloalkyle-condensé et qui peut contenir éventuellement un ou plusieurs hétéroatomes supplémentaires choisis parmi O et N,

sous la réserve que si X et Y sont tous les deux O, m est 1 et $-NR^2R^3$ est autre qu'un hetérocycle non saturé, substitué ou non substitué, qui peut être éventuellement aryle-condensé ou cycloalkyle-condensé et qui peut contenir éventuellement un ou plusieurs hétéroatomes supplémentaires choisis parmi O et N, $(R^1)_n$ ne peut alors pas être un substituant unique (n = 1) en position 4 de l'anneau cyclohexane et ayant la formule

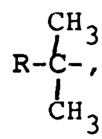

dans laquelle R représente un atome d'hydrogène, un groupe alkyle ou un groupe phényle éventuellement substitué,

et sous la réserve supplémentaire que si X et Y sont tous les deux O, m est 1 et $-NR^2R^3$ est un groupe amino ou phtalimido, $(R^1)_n$ ne peut alors pas être seulement 8-méthyle, 8-méthoxy ou 6-phényle ou seulement 7,7,9,9-tetraméthyle, caractérisé en ce qu'on fait réagir un cétal de formule générale II

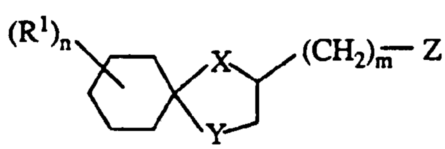

II

dans laquelle X, Y, $(R^1)_n$ et m ont les significations définies ci-dessus, et dans laquelle Z représente un groupe échangeable attirant les électrons, avec une amine de formule générale III

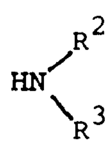

III

dans laquelle $R^2$ et $R^3$ ont les significations définies ci-dessus, éventuellement en présence d'un diluant et éventuellement en présence d'un catalyseur, et éventuellement en présence d'un fixateur d'acide en surplus d'un excédent éventuel de l'amine III.

2. Agent fongicide, particulièrement agent fongicide sur plantes, et plus particulièrement agent fongicide sur céréales, pour la lutte contre le mildiou, la rouille et autres maladies significatives de feuillages, caractérisé en ce qu'il contient, comme composant actif, au moins un composé choisi parmi les aminocétals de formule I

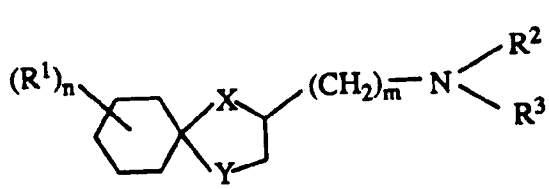

I

dans laquelle X et Y représentent chacun, indépendamment l'un de l'autre, O ou $-OCH_2-$, $(R^1)_n$ représente jusqu'à quatre substituants identiques ou différents choisis parmi des groupes alkyle à chaîne droite ou ramifiée et contenant jusqu'à 10 atomes de carbone, cycloalkyle contenant jusqu'à 6 atomes de carbone, aryle, aryloxy, alcoxy, cycloalcoxy, arylalkyle et alkythio, deux substituants $R^1$ voisins pouvant aussi former conjointement un anneau saturé ou non saturé, et

n est 1, 2, 3 ou 4, et m est 0, 1, 2, 3 ou 4, et

$R^2$ et $R^3$ représentent chacun, indépendamment l'un de l'autre, un atome d'hydrogène, un groupe alkyle à chaîne droite ou ramifiée et contenant jusqu'à 10 atomes de carbone, un groupe cycloalkyle non substitué ou substitué par alkyle, un groupe alcényle, un groupe alcynyle ou un groupe arylalkyle non substitué ou substitué par alkyle, ou

$R^2$ et $R^3$ représentent, conjointement avec l'atome d'azote auquel ils sont liés, un hétérocycle saturé ou non saturé, substitué ou non substitué, qui peut être éventuellement aryle-condensé ou cycloalkyle-condensé et qui peut contenir éventuellement un ou plusieurs hétéroatomes supplémentaires choisis parmi O et N,

sous la réserve que si X et Y sont tous les deux O, m est 1 et $-NR^2R^3$ est autre qu'un hétérocycle non saturé, substitué ou non substitué, qui peut être éventuellement aryle-condensé ou cycloalkyle-condensé et qui peut contenir éventuellement un ou plusieurs hétéroatomes supplémentaires choisis parmi O et N, $(R^1)_n$ ne peut alors pas être un substituant unique (n = 1) en position 4 de l'anneau cyclohexane et ayant la formule

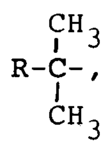

dans laquelle R représente un atome d'hydrogène, un groupe alkyle ou un groupe phényle éventuellement substitué,

et sous la réserve supplémentaire que si X et Y sont tous les deux O, m est 1 et $-NR^2R^3$ est un groupe amino ou phtalimido, $(R^1)_n$ ne peut alors pas être seulement 8-méthyle, 8-méthoxy ou 6-phényle ou seulement 7,7,9,9-tetraméthyle.

3. Utilisation d'un aminocétal de formule I selon la revendication 2 pour la lutte contre champignons, particulièrement champignons phytopathogènes, et plus particulièrement le mildiou, la rouille et autres maladies significatives des feuillages de céréales.

4. Procédé pour la lutte contre des champignons, particulièrement champignons phytopathogènes, et plus particulièrement le mildiou, la rouille et autres maladies significatives des feuillages de céréales, caractérisé en ce qu'on laisse agir un aminocétal de formule I selon la revendication 2 sur les champignons concernés et/ou dans leur biotope.

5. Procédé pour la préparation d'un agent fongicide, particulièrement d'un agent fongicide sur plantes, et plus particulièrement d'un agent fongicide sur céréales, pour la lutte contre le mildiou, la rouille et autres maladies significatives des feuillages, caractérisé en ce qu'on mélange un aminocétal de formule I selon la revendication 1 avec un ou plusieurs agents choisis parmi des charges, des agents tensioactifs et d'autres agents auxiliaires usuels.